(19) Europäisches Patentamt
European Patent Office
Office européen des brevets

(11) EP 2 791 291 B1

(12) EUROPÄISCHE PATENTSCHRIFT

(45) Veröffentlichungstag und Bekanntmachung des Hinweises auf die Patenterteilung:
**26.04.2023 Patentblatt 2023/17**

(21) Anmeldenummer: **12799567.8**

(22) Anmeldetag: **12.12.2012**

(51) Internationale Patentklassifikation (IPC):
***C10M 133/02*** (2006.01) ***C10M 133/06*** (2006.01)
***C10M 133/54*** (2006.01) ***C07C 209/20*** (2006.01)
***C07C 211/63*** (2006.01) ***C10L 1/22*** (2006.01)
***C10L 10/04*** (2006.01) ***C10L 10/06*** (2006.01)
***C10N 40/25*** (2006.01) ***C10N 30/04*** (2006.01)
***C10N 70/00*** (2006.01)

(52) Gemeinsame Patentklassifikation (CPC):
**C10M 133/06; C07C 209/20; C07C 211/63; C10L 1/2222; C10L 10/06; C10L 10/18; C10M 133/02; C10M 133/54;** C10L 1/14; C10L 2270/023; C10L 2270/026; C10M 2215/02; C10M 2215/04; C10M 2215/042; C10M 2215/26;

(Forts.)

(86) Internationale Anmeldenummer:
**PCT/EP2012/075244**

(87) Internationale Veröffentlichungsnummer:
**WO 2013/087701 (20.06.2013 Gazette 2013/25)**

(54) **VERWENDUNG QUATERNISIERTER ALKYLAMINE ALS ADDITIVE IN KRAFT- UND SCHMIERSTOFFEN**

USE OF QUATERNISED ALKYLAMINE AS ADDITIVE IN FUELS AND LUBRICANTS

UTILISATION D'ALKYLAMINE QUATERNISÉ COMME ADDITIF DANS DES CARBURANTS ET DES LUBRIFIANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.12.2011 EP 11193034**

(43) Veröffentlichungstag der Anmeldung:
**22.10.2014 Patentblatt 2014/43**

(60) Teilanmeldung:
**23158753.6**

(73) Patentinhaber: **BASF SE**
**67056 Ludwigshafen am Rhein (DE)**

(72) Erfinder:
- **HANSCH, Markus**
**67346 Speyer (DE)**
- **BÖHNKE, Harald**
**68167 Mannheim (DE)**
- **VÖLKEL, Ludwig**
**67117 Limburgerhof (DE)**
- **GRABARSE, Wolfgang**
**68163 Mannheim (DE)**
- **STRITTMATTER, Jan**
**Shanghai 200030 (CN)**

(74) Vertreter: **BASF IP Association**
**BASF SE**
**GBI-C006**
**67056 Ludwigshafen (DE)**

(56) Entgegenhaltungen:
**EP-A1- 0 293 192** **EP-A1- 0 345 475**
**EP-A1- 2 033 945** **EP-A2- 0 189 085**
**WO-A1-2013/070503** **WO-A2-2006/135881**
**US-A- 4 902 437** **US-A1- 2008 141 580**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 791 291 B1

(52) Gemeinsame Patentklassifikation (CPC): (Forts.)
C10N 2030/04; C10N 2030/54; C10N 2040/252;
C10N 2040/255; C10N 2070/02; Y02T 10/12

## Beschreibung

Die vorliegende Erfindung betrifft die Verwendung quaternisierter Alkylamin-Stickstoffverbindungen als Kraft- und Schmierstoffadditiv, wie insbesondere als Detergensadditiv; zur Verringerung oder Verhinderung von Ablagerungen in den Einspritzsystemen von direkteinspritzenden Dieselmotoren, insbesondere in Common-Rail-Einspritzsystemen, zur Verringerung des Kraftstoffverbrauches von direkteinspritzenden Dieselmotoren, insbesondere von Dieselmotoren mit Common-Rail-Einspritzsystemen, und zur Minimierung des Leistungsverlustes (power loss) in direkteinspritzenden Dieselmotoren, insbesondere in Dieselmotoren mit Common-Rail-Einspritzsystemen; sowie als Additiv für Ottokraftstoffe, insbesondere zum Betrieb von DISI Motoren.

## Stand der Technik:

Bei direkteinspritzenden Dieselmotoren wird der Kraftstoff durch eine direkt in den Brennraum des Motors reichende Mehrloch-Einspritzdüse eingespritzt und feinst verteilt (vernebelt), anstatt wie beim klassischen (Kammer-) Dieselmotor in eine Vor- oder Wirbelkammer eingeführt zu werden. Der Vorteil der direkteinspritzenden Dieselmotoren liegt in ihrer für Dieselmotoren hohen Leistung und einem dennoch geringen Verbrauch. Außerdem erreichen diese Motoren ein sehr hohes Drehmoment schon bei niedrigen Drehzahlen.

Zurzeit werden im Wesentlichen drei Verfahren eingesetzt, um den Kraftstoff direkt in den Brennraum des Dieselmotors einzuspritzen: die konventionelle Verteilereinspritzpumpe, das Pumpe-Düse-System (Unit-Injector-System bzw. Unit-Pump-System) und das Common-Rail-System.

Beim Common-Rail-System wird der Dieselkraftstoff von einer Pumpe mit Drücken bis zu 2000 bar in eine Hochdruckleitung, die Common-Rail gefördert. Ausgehend von der Common-Rail laufen Stichleitungen zu den verschiedenen Injektoren, die den Kraftstoff direkt in den Brennraum injizieren. Dabei liegt auf der Common-Rail stets der volle Druck an, was eine Mehrfacheinspritzung oder eine spezielle Einspritzform ermöglicht. Bei den anderen Injektionssystemen ist dagegen nur eine geringere Variation der Einspritzung möglich. Die Einspritzung beim Common-Rail wird im Wesentlichen in drei Gruppen unterteilt: (1.) Voreinspritzung, durch die im Wesentlichen eine weichere Verbrennung erreicht wird, so dass harte Verbrennungsgeräusche (" Nageln" ) vermindert werden und der Motorlauf ruhig erscheint; (2.) Haupteinspritzung, die insbesondere für einen guten Drehmomentverlauf verantwortlich ist; und (3.) Nacheinspritzung, die insbesondere für einen geringen $NO_x$-Wert sorgt. Bei dieser Nacheinspritzung wird der Kraftstoff in der Regel nicht verbrannt, sondern durch Restwärme im Zylinder verdampft. Das dabei gebildete Abgas-/Kraftstoffgemisch wird zur Abgasanlage transportiert, wo der Kraftstoff in Gegenwart geeigneter Katalysatoren als Reduktionsmittel für die Stickoxide $NO_x$ wirkt.

Durch die variable, zylinderindividuelle Einspritzung kann beim Common-Rail-Einspritzsystem der Schadstoffausstoß des Motors, z.B. der Ausstoß von Stickoxiden ($NO_x$), Kohlenmonoxid (CO) und insbesondere von Partikeln (Ruß), positiv beeinflusst werden. Dies ermöglicht beispielsweise, dass mit Common-Rail-Einspritzsystemen ausgerüstete Motoren der Euro 4-Norm theoretisch auch ohne zusätzlichen Partikelfilter genügen können.

In modernen Common-Rail-Dieselmotoren können sich unter bestimmten Bedingungen, beispielsweise bei Verwendung von biodieselhaltigen Kraftstoffen oder von Kraftstoffen mit Metall-Verunreinigungen wie Zink-Verbindungen, Kupfer-Verbindungen, Bleiverbindungen und weiteren Metallverbindungen, an den Injektoröffnungen Ablagerungen bilden, die das Einspritzverhalten des Kraftstoffs negativ beeinflussen und dadurch die Performance des Motors beeinträchtigen, d.h. insbesondere die Leistung verringern, aber zum Teil auch die Verbrennung verschlechtern. Die Bildung von Ablagerungen wird durch bauliche Weiterentwicklungen der Injektoren, insbesondere durch die Veränderung der Geometrie der Düsen (engere, konische Öffnungen mit abgerundetem Auslass) noch verstärkt. Für eine dauerhaft optimale Funktionsweise von Motor und Injektoren müssen solche Ablagerungen in den Düsenöffnungen durch geeignete Kraftstoffadditive verhindert oder reduziert werden

In den Einspritzsystemen modernen Dieselmotoren verursachen Ablagerungen signifikante Performance-Probleme. Weit verbreitet ist die Erkenntnis, dass derartige Ablagerungen in den Sprühkanälen zu einer Verringerung des Kraftstoffflusses und damit zu Leistungsverlusten (power loss) führen können. Ablagerungen an der Injektorspitze beeinträchtigen dagegen die optimale Ausbildung von Kraftstoff-Sprühnebel und bedingen dadurch eine verschlechterte Verbrennung und damit verbunden höhere Emissionen und vermehrten Kraftstoffverbrauch. Im Gegensatz zu diesen herkömmlichen, " äußeren" Ablagerungsphänomenen bereiten auch " interne" Ablagerungen (zusammengefasst als innere Diesel-Injektor-Ablagerungen (IDID)) in bestimmten Teilen der Injektoren, wie an der Düsennadel, am Steuerkolben, am Ventilkolben, am Ventilsitz, an der Ansteuereinheit und an den Führungen dieser Komponenten zunehmend Performance-Probleme. Herkömmliche Additive zeigen eine unzureichende Wirkung gegen diese IDIDs.

Aus der US 4,248,719 sind quaternisierte Ammoniumsalze beschrieben, welche durch Umsetzung eines Alkenylsuccinimids mit einem Monocarbonsäureester hergestellt werden und als Dispergiermittel in Schmierölen zur Verhinderung von Schlammbildung Anwendung finden. Insbesondere ist beispielsweise die Umsetzung von Polyisobutylbernsteinsäureanhydrid (PIBSA) mit N,N-Dimethylaminopropylamin (DMAPA) und Quaternisierung mit Methylsalicylat

beschrieben. Eine Anwendung in Kraftstoffen, insbesondere Dieselkraftstoffen, wird darin jedoch nicht vorgeschlagen. Die Verwendung von PIBSA mit niedrigen Bismaleinierungsgraden < 20 % wird darin nicht beschrieben.

Aus der US 4,171,959 sind quaternisierte Ammoniumsalze von hydrocarbylsubstituierten Succinimiden beschrieben, welche als Detergenzadditive für Ottokraftstoffzusammensetzungen geeignet sind. Zur Quaternisierung werden bevorzugt Alkylhalogenide eingesetzt. Erwähnt sind außerdem organische $C_2$-$C_8$-Hydrocarbyl-Carboxylate und -Sulfonate. Folglich weisen die gemäß dortiger Lehre bereitgestellten quaternisierten Ammoniumsalze als Gegenion entweder ein Halogenid oder ein $C_2$-$C_8$-Hydrocarbyl-Carboxylat oder ein $C_2$-$C_8$-Hydrocarbyl-Sulfonat-Gruppe auf. Die Verwendung von PIBSA mit niedrigen Bismaleinierungsgraden <20 % wird darin ebenfalls nicht beschrieben.

Aus der EP-A-2 033 945 sind Kaltfließverbesserer bekannt, welche durch Quaternisierung spezieller tertiärer Monoamine, die wenigstens einen $C_8$-$C_{40}$-Alkylrest tragen, mit einem $C_1$-$C_4$-Alkylester spezieller Carbonsäuren hergestellt werden. Beispiele solcher Carbonsäureester sind Dimethyloxalat, Dimethylmaleat, Dimethylphthalat und Dimethylfumarat. Andere Anwendungen als zur Verbesserung des CFPP Werts von Mitteldestillaten sind in der EP-A-2 033 945 nicht belegt.

Die WO 2006/135881 beschreibt quaternisierte Ammoniumsalze, hergestellt durch Kondensation eines Hydrocarbyl-substituierten Acylierungsmittels und einer Sauerstoff- oder Stickstoffatom-haltigen Verbindung mit tertiärer Aminogruppe, und anschließender Quaternisierung mittels Hydrocarbylepoxid in Kombination mit stöchiometrischen Mengen einer Säure, wie insbesondere Essigsäure. Weitere in der WO 2006/135881 beanspruchte Quaternisierungsmittel sind Dialkylsulfate, Benzylhalogenide und hydrocarbylsubstituierte Carbonate, wobei Dimethylsulfat, Benzylchlorid und Dimethylcarbonat experimentell untersucht wurden.

Die in der WO 2006/135881 bevorzugt verwendeten Quaternisierungsmittel weisen jedoch gravierende Nachteile auf, wie: Toxizität bzw. Carcinogenität (z.B. bei Dimethylsulfat und Benzylhalogeniden), keine rückstandsfreie Verbrennung (z.B. bei Dimethylsulfat und Alkylhalogeniden), sowie unzureichende Reaktivität, welche zu unvollständiger Quaternierung oder nicht-ökonomischen Reaktionsbedingungen (lange Reaktionszeiten, hohe Reaktionstemperaturen, Überschuss an Quaternierungsmittel; z.B. bei Dimethylcarbonat) führt.

Die EP-A-2 033 945 beschreibt die Herstellung halogen- und schwefelfreier quaternärer Ammoniumsalze von organischen Carbonsäuren (wie z.B. Oxalsäure, Phthalsäure, Salicylsäure, Malonsäure und Maleinsäure sowie deren Alkylester) und deren Verwendung zur Verbesserung des CFPP-Werts von Dieselkraftstoffen.

Quaternäre Ammoniumsalze von alpha-Hydroxycarbonsäuren werden in der EP-A-1 254 889 als Reinigungsmittel für elektronische Bauteile vorgeschlagen.

Weiterhin beschreibt die Japanische Patentanmeldung, Anmeldenummer 61-012197, die Verwendung quaternärer Ammoniumsalze von organischen Carbonsäuren als Oberflächenaktives mittel oder Rohmaterial für Medikamente oder Kosmetika.

Es bestand daher die Aufgabe, weitere Kraftstoffadditive bereitzustellen, welche Ablagerungen in der Injektorspitze und interne Injektorablagerungen beim Betrieb von Common-Rail-Dieselmotoren verhindern.

## Kurze Beschreibung der Erfindung:

Es wurde nun überraschenderweise gefunden, dass obige Aufgabe durch Bereitstellung quaternisierter Kohlenwasserstoffaminverbindungen (wie Hydrocarbylaminverbindungen) bzw. damit additivierter Kraft- und Schmierstoffzusammensetzungen gelöst wird.

Überraschenderweise sind die erfindungsgemäßen Additive wie insbesondere durch die beiliegenden Anwendungsbeispiele veranschaulicht, überraschend wirksam in Common-Rail-Dieselmotoren und zeichnen sich durch ihre besondere Eignung als Additiv zur Verringerung von Powerloss durch externe und Kaltstartprobleme durch interne Ablagerungen aus.

## Figurenbeschreibung:

Figur 1 zeigt eine Messung der zeitabhängigen (h) Veränderung der Abgastemperaturen der Zylinder bei Verwendung eines Kraftstoffs ohne Additiv; hohe Abweichungen der Temperatur werden durch interne Injektorablagerungen verursacht.

Figur 2 zeigt die zeitabhängigen (h) Veränderung der Abgastemperaturen derselben Zylinder wie in Figur 1, nun jedoch nach Behandlung mit dem erfindungsgemäßen Additiv von Herstellbeispiel 3, Dosierung 394 mg/kg.

Figur 3 zeigt den Ablauf eines einstündigen Motorentestzyklus gemäß CEC F-098-08.

## Detaillierte Beschreibung der Erfindung:

### A1) Spezielle Ausführungsformen

Die vorliegende Erfindung betrifft insbesondere folgende spezielle Ausführungsformen:
Eine erste Ausführungsform der Erfindung betrifft die Verwendung eines eine quaternisierte Stickstoffverbindung umfassenden Reaktionsprodukts oder einer aus dem Reaktionsprodukt durch Aufreinigung erhaltene, eine quaternisierte Stickstoffverbindung enthaltende Teilfraktion davon, wobei das Reaktionsprodukt erhältlich ist durch Umsetzung eines quaternisierbaren Alkylamins, enthaltend wenigstens eine quaternisierbare, tertiäre Aminogruppe mit einem Quaternierungsmittel, das die wenigstens eine tertiäre Aminogruppe in eine quaternäre Ammoniumgruppe überführt,
wobei das Quaternierungsmittel eine Verbindung der allgemeinen Formel 1 ist,

$$R_1OC(O)R_2 \qquad (1)$$

worin

$R_1$ für einen gesättigten, geradkettigen oder verzweigten Kohlenwasserstoffrest mit 1 bis 4 Kohlenstoffatomen, wie insbesondere Methyl oder Ethyl, steht und
$R_2$ für einen gegebenenfalls substituierten einkernigen Aryl- oder Cycloalkylrest, insbesondere Aryl, wie Phenyl, steht, wobei der Substituent ausgewählt ist unter OH, $NH_2$, $NO_2$, $C(O)OR_3$ und $R_1OC(O)$-; worin $R_1$ die oben angegebenen Bedeutungen besitzt und $R_3$ für H oder $R_1$ steht; wobei der Substituent insbesondere OH ist;
oder $R_2$ für $R_{1a}OC(O)$- steht, worin $R_{1a}$ die oben für $R_1$ angegebenen Bedeutungen besitzt; oder
wobei das Quaternierungsmittel eine Verbindung der allgemeinen Formel 2 ist,

$$R_1OC(O)\text{-}A\text{-}C(O)OR_{1a} \qquad (2)$$

worin
$R_1$ und $R_{1a}$ unabhängig voneinander für einen gesättigten, geradkettigen oder verzweigten Kohlenwasserstoffrest mit 1 bis 4 Kohlenstoffatomen steht und
A für eine gegebenenfalls ein- oder mehrfach substituierte geradkettige oder ein- oder mehrfach verzweigte Kohlenwasserstoffbrückengruppe mit 1-10 Kohlenstoffatomen steht, welche gegebenenfalls durch ein oder mehrere Heteroatomgruppen unterbrochen ist; wobei die Substituenten beispielsweiseausgewählt sind unter OH, $NH_2$, $NO_2$, oder $C(O)OR_3$, insbesondere OH und $C(O)OR_3$, wobei $R_3$ wie oben definiert ist;
oder
wobei das Quaternisierungsmittel ein Epoxid der allgemeinen Formel 4 ist,

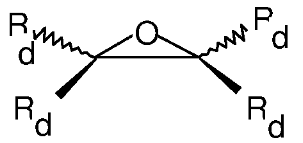 (4)

wobei
die darin enthaltenen Reste $R_d$ gleich oder verschieden sind und für H oder für einen aliphatischen oder aromatischen Rest mit wenigstens 1 bis 10 Kohlenstoffatomen stehen, wobei die aliphatischen Reste $R_d$ zusätzlich Heteroatome, wie O, N, NH, S, in ihrer Kette enthalten können; alleine oder in Kombination mit einer freien Säure umfasst; oder
wobei das Quaternisierungsmittel ein Dialkylcarbonat ist;
und wobei
das Alkylamin wenigstens eine Verbindung der folgenden allgemeinen Formel 3 umfasst,

$$R_aR_bR_cN \qquad (3)$$

worin
wenigstens einer der Reste $R_a$, $R_b$ und $R_c$, , wie z.B. einer oder zwei, für einen geradkettigen oder verzweigte, gesättigten oder ungesättigten $C_8$-$C_{40}$-Kohlenwasserstoffrest, welcher keine Heteroatome aufweist, steht und die übrigen Reste für gleiche oder verschiedene, geradkettige oder verzweigte, gesättigte oder ungesättigte $C_1$-$C_6$-Kohlenwasserstoffreste stehen; oder
worin alle Reste $R_a$, $R_b$ und $R_c$ für gleiche oder verschiedene geradkettige oder verzweigte, gesättigte oder unge-

sättigte $C_8$-$C_{40}$-Kohlenwasserstoffreste, welche keine Heteroatome aufweisen, insbesondere $C_8$ - $C_{40}$-Alkyl-Reste stehen.

als Dieselkraftstoffadditiv zur Verringerung und/oder Vermeidung von Internal Diesel Injector Deposits (IDID)in direkteinspritzenden Dieselmotoren.

Insbesondere ist das Quaternisierungsmittel ein Phthalat oder ein Salicylat, wie Dimethylphthalat, oder Methylsalicylat

In einer spezieller Ausführungsform ist das quaternisierbare tertiäre Amin eine Verbindung der Formel 3, worin wenigstens zwei der Reste $R_3$ ,$R_b$ und $R_c$ gleich oder verschieden sind und für einen geradkettigen oder verzweigte, $C_{10}$-$C_{20}$-Alkylrest steht und der übrigen Rest für $C_1$-$C_4$-Alkyl stehen.

In einer weiterer spezieller Ausführungsform ist das das Quaternierungsmittel ausgewählt unter Niedrigalkylenoxiden in Kombination mit einer Monocarbonsäure, Alkylsalicylaten, Dialkylphthalaten und Dialkyloxalaten.

In einer weiterer spezieller Ausführungsform erfolgt die Anwendung in Dieselkraftstoffen, Biodieselkraftstoffen, wie Bioethanol-haltigen Dieselkraftstoffe, insbesondere Dieselkraftstoffen.

Die erfindungsgemäße Verwendung dient als Dieselkraftstoffadditiv zur Verringerung und/oder Vermeidung von der Internal Diesel Injector Deposits (IDID) in direkteinspritzenden Dieselmotoren, insbesondere in Common-Rail-Einspritzsystemen (wie z.B. bestimmt nach einer IDID Testprozedur, wie unten im experimentellen Teil näher beschrieben).

Jeweils geeignete Testmethoden zur Überprüfung der oben bezeichneten Anwendungen sind den Fachmann bekannt, bzw. in folgenden experimentellen Teil, worauf hiermit ausdrücklich allgemein Bezug genommen wird, beschrieben.

**A2) Allgemeine Definitionen**

Werden keine gegenteiligen Angaben gemacht, so gelten folgende allgemeine Bedeutungen:
Der Begriff " Kohlenwasserstoffrest" ist breit auszulegen und umfasst sowohl langkettige als auch kurzkettige, gerade oder verzweigte Kohlenwasserstoffreste mit 1 bis 50 Kohlenstoffatomen, welche ggf. zusätzlich Heteroatome, wie z.B. O, N, NH,S, in ihrer Kette enthalten können. Eine spezielle Gruppe von Kohlenwasserstoffresten umfasst sowohl langkettige als auch kurzkettige, geradkettige oder verzweigte Alkylreste mit 1 bis 50 Kohlenstoffatomen.

" Langkettige" Kohlenwasserstoffrestreste stehen für geradkettige oder verzweigte Kohlenwasserstoffreste und weisen 7 bis 50 oder 8 bis 50 oder 8 bis 40 oder 10 bis 20 Kohlenstoffatomen, welche ggf. zusätzlich Heteroatome, wie z.B. O, N, NH,S, in ihrer Kette enthalten können. Weiterhin können die Reste ein- oder mehrfach ungesättigt sein und ein oder mehrere nicht-kumulierte, wie z.B. 1 bis 5, wie 1, 2 oder 3 C-C Doppelbindungen oder C-C- Dreifachbindungen, insbesondere 1, 2 oder 3 Doppelbindungen, aufweisen. Sie können natürlichen oder synthetischen Ursprungs sein. Sie können auch ein zahlengemitteltes Molekulargewicht ($M_n$) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500 aufweisen Sie sind dann insbesondere im Wesentlichen aus $C_{2-6}$-, insbesondere $C_{2-4}$-Monomerbausteinen, wie Ethylen, Propylen, n- oder iso-Butylen oder Mischungen davon aufgebaut, wobei die verschiedenen Monomere statistisch verteilt oder als Blöcke einpolymerisiert enthalten sein können. Derartige langkettige Kohlenwasserstoffrestreste werden auch als Polyalkylenreste oder Poly-$C_{2-6}$- oder Poly-$C_{2-4}$-alkylenreste bezeichnet. Geeignete langkettige Kohlenwasserstoffrestreste und deren Herstellung sind beispielsweise auch beschreiben in der WO2006/135881 und der dort zitierten Literatur. Eine spezielle Gruppe von langkettigen Kohlenwasserstoffresten umfasst geradkettige oder verzweigte Alkylresten (" langkettige" Alkylreste" ) mit 8 bis 50, wie z.B. 8 bis 40 oder 8 bis 30 oder 10 bis 20 Kohlenstoffatomen.

" Kurzkettiges Kohlenwasserstoffrest" oder " niedermolekulares Kohlenwasserstoffrest" steht insbesondere für geradkettiges oder verzweigtes Alkyl oder Alkenyl, gegebenenfalls unterbrochen durch eine oder mehrere, wie z.B. 2, 3 oder 4 Heteroatomgruppen, wie -O- oder - NH-. oder gegebenenfalls ein- oder mehrfach, wie z.B. 2, 3 oder 4-fach substituiert.

" Kohlenwasserstoffbrückengruppe" steht für geradkettige oder ein- oder mehrfach verzweigte Brückengruppen mit 1 bis 10 Kohlenstoffatomen, gegebenenfalls unterbrochen durch eine oder mehrere, wie z.B. 2, 3 oder 4 Heteroatomgruppen, wie -O- oder - NH-. oder gegebenenfalls ein- oder mehrfach, wie z.B. 2, 3 oder 4-fach substituiert.

" Alkyl" oder " Niedrigalkyl" steht insbesondere für gesättigte, geradkettige oder verzweigte Kohlenwasserstoffreste mit 1 bis 4, 1 bis 5, 1 bis 6, oder 1 bis 7, Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, sowie die ein- oder mehrfach verzweigten Analoga davon.

" Langkettiges Alkyl" steht insbesondere für gesättigte, geradkettige oder verzweigte Kohlenwasserstoffreste

mit 8 bis 50, wie z.B. 8 bis 40 oder 8 bis 30 oder 10 bis 20 Kohlenstoffatomen, wie Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Hencosyl, Docosyl, Tricosyl, Tetracosyl, Pentacosyl, Hexacosyl, Heptacosyl, Octacosyl, Nonacosyl, Squalyl, Konstitutionsisomere, insbesondere ein oder mehrfach verzweigte Isomere und höhere Homologe davon.

" Alkenyl" steht für ein- oder mehrfach, insbesondere einfach ungesättigte, geradkettige oder verzweigte Kohlenwasserstoffreste mit 2 bis 4, 2 bis 6, oder 2 bis 7 Kohlenstoffatomen und einer Doppelbindung in einer beliebigen Position, z. B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

" Alkylen" steht für geradkettige oder ein- oder mehrfach verzweigte Kohlenwasserstoff-Brückengruppen mit 1 bis 10 Kohlenstoffatomen, wie z.B. $C_1$-$C_7$-Alkylengruppen ausgewählt unter $-CH_2-$, $-(CH_2)_2-$, $-(CH_2)_3-$, $-CH_2-CH(CH_3)-$, $-CH(CH_3)-CH_2-$, $-(CH_2)_4-$, $-(CH_2)_2-CH(CH_3)-$, $-CH_2-CH(CH_3)-CH_2-$, $(CH_2)_4-$, $-(CH_2)_5-$, $-(CH_2)_6$, $-(CH_2)_7-$, $-CH(CH_3)-CH_2-CH_2-CH(CH_3)-$ oder $-CH(CH_3)-CH_2-CH_2-CH_2-CH(CH_3)-$ oder $C_1$-$C_4$-Alkylengruppen ausgewählt unter $-CH_2-$, $-(CH_2)_2-$, $-(CH_2)_3-$, $-CH_2-CH(CH_3)-$, $-CH(CH_3)-CH_2-$, $-(CH_2)_4-$, $-(CH_2)_2-CH(CH_3)-$, $-CH_2-CH(CH_3)-CH_2-$,

" Alkenylen" steht für die ein- oder mehrfach, insbesondere einfach ungesättigten Analoga obiger Alkylengruppen mit 2 bis 10 Kohlenstoffatomen, insbesondere für $C_2$-$C_7$-Alkenylene oder $C_2$-$C_4$-Alkenylen, wie -CH=CH-, $-CH=CH-CH_2-$, $-CH_2-CH=CH-$, $-CH=CH-CH_2-CH_2-$, $-CH_2-CH=CH-CH_2-$, $-CH_2-CH_2-CH=CH-$, $-CH(CH_3)-CH=CH-$, $-CH_2-C(CH_3)=CH-$,

" Cycloalkyl" steht für carbocyclische Reste mit 3 bis 20 Kohlenstoffatomen, wie z.B. C3-C12-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder C3-C7-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann.

-"Aryl" steht für ein- oder mehrkernige, vorzugsweise ein- oder zweikernige, gegebenenfalls substituierte aromatische Reste mit 6 bis 20 wie z.B. 6 bis 10 RingKohlenstoffatomen, wie z.B. Phenyl, Biphenyl, Naphthyl wie 1- oder 2-Naphthyl, Tetrahydronaphthyl, Fluorenyl, Indenyl und Phenathrenyl. Diese Arylreste können gegebenenfalls 1, 2, 3, 4, 5 oder 6 gleiche oder verschiedene Substituenten tragen.

" Substituenten" für hierin angegebene Reste, sind insbesondere, wenn keine anderen Angaben gemacht werde, ausgewählt sind unter Ketogruppen, - COOH, -COO-Alkyl, - OH, -SH, -CN, Amino, $-NO_2$, Alkyl, oder Alkenylgruppen.

" Mn" steht für das zahlenmittleres Molekulargewicht und wird in herkömmlicher Weise bestimmt; insbesondere beziehen sich solche Angaben auf Mn-Werte, bestimmt durch Gelpermeationschromatographie.

### A3) Tertiäre Amine der Formel (3)

Tertiäre Amine der Formel (3) sind an sich bekannte Verbindungen, wie z.B. beschrieben in der EP-A-2 033 945.

Das tertiäre Amin-Edukt (3) trägt vorzugsweise ein Segment der Formel $NR_a R_b$, wobei einer der Reste eine Alkylgruppe mit 8 bis 40 Kohlenstoffatomen und der andere eine Alkylgruppe mit bis zu 40, besonders bevorzugt 8 bis 40 Kohlenstoffatomen aufweist. Der Rest $R_c$ ist dabei insbesondere ein kurzkettiger $C_1$-$C_6$-Alkylrest, wie eine Methyl-, Ethyl- oder Propyl-Gruppe. $R_a$ und $R_b$ können geradkettig oder verzweigt sein, und / oder können gleich oder verschieden sein. Beispielsweise können $R_a$ und $R_b$ eine geradkettige $C_{12}$-$C_{24}$-Alkylgruppe sein. Alternativ kann nur einer der beiden Reste langkettig (z.B. mit mit 8 bis 40 Kohlenstoffatomen) sein und der andere eine Methyl-, Ethyl- oder Propyl-Gruppe darstellen.

Zweckmäßig ist das Segment $NR_aR_b$ von einem sekundären Amin abgeleitet, wie Dioctadecylamin, Di-Kokosamin, di-hydriertes Talgamin und Methylbehenylamin. Amingemische, wie sie aus natürlichen Materialien erhältlich sind, eignen sich ebenfalls. Beispielsweise ist zu nennen ein sekundäres hydriertes Talg-Amin, wobei die Alkylgruppen

von hydriertem Talgfett abgeleitet sind, und etwa 4 Gew.-% $C_{14}$, 31 Gew.-% $C_{16}$ und 59 Gew.-% $C_{18}$-Alkylgruppen aufweisen. Entsprechende tertiäre Amine der Formel (3) werden beispielsweise von der Firma Akzo Nobel unter der Bezeichnung Armeen® M2HT oder Armeen® M2C vertrieben.

Das tertiäre Amin-Edukt (3) kann aber auch so ausgebildet sein, dass die Reste $R_a$, $R_b$ und $R_c$ gleich oder verschiedene langkettige Alkylreste insbesondere geradkettige oder verzweigte Alkylgruppen mit 8 bis 40 Kohlenstoffatomen aufweisen.

Weitere nichtlimitierende Beispiele für geeignete Amine sind:
N,N-Dimethyl-N-(2-ethylhexyl)amin, N,N-Dimethyl-N-(2-propylheptyl)amin, Dodecyldimethylamin, Hexadecyldimethylamin, Oleyldimethylamin, Cocoyldimethylamin, Dicocoylmethylamin, Talgfettdimethylamin, Ditalgfettmethylamin, Tridodecylamin, Trihexadecylamin, Trioctadecylamin, Sojadimethylamin, Tris(2-ethylhexyl)amin, sowie Alamine 336 (Tri-n-octylamin).

**A4) Quatemislerungsmittel:**

Als Quaternisierungsmittel kommen im Prinzip alle als solche geeigneten Verbindungen in Betracht. Das Quaternisierungsmittel ist insbesondere ausgewählt unter Alkylenoxiden ggf. in Kombination mit Säure; aliphatische oder aromatische Carbonsäureestern, wie insbesondere Dialkylcarboxylaten; Alkanoaten; cyclischen nichtaromatischen oder aromatischen Carbonsäureestern; Alkylsulfaten; Alkylhalogeniden; Alkylarylhalogeniden; Dialkylcarbonate; und Mischungen davon.

Geeignet sind z.B. Alkylester, abgeleitet von Carbonsäuren, deren $pK_s$-Wert kleiner als 3,5 ist. Beispiele sind insbesondere Alkylester abgeleitet von Oxalsäure, Phthalsäure, Salicylsäure, Maleinsäure, Malonsäure und Citronensäure,

In einer besonderen Ausführungsform erfolgt die Quaternisierung des mindestens einen quaternisierbaren tertiären Stickstoffatoms jedoch mit mindestens einem Quaternisierungsmittel ausgewählt unter

a) Verbindungen der allgemeinen Formel 1 ist

$$R_1OC(O)R_2 \qquad (1)$$

worin

$R_1$ für einen gesättigten, geradkettigen oder verzweigten Kohlenwasserstoffrest mit 1 bis 4 Kohlenstoffatomen, steht und
$R_2$ für einen gegebenenfalls substituierten einkernigen Aryl- oder Cycloalkylrest, steht, wobei der Substituent ausgewählt ist unter OH, $NH_2$, $NO_2$, $C(O)OR_3$; $R_{1a}OC(O)$-, worin $R_{1a}$ die oben für $R_1$ angegebenen Bedeutungen besitzt, und $R_3$ für H oder $R_1$ steht;
oder

b) Verbindungen der allgemeinen Formel 2

$$R_1OC(O)\text{-}A\text{-}C(O)OR_{1a} \qquad (2)$$

worin

$R_1$ und $R_{1a}$ unabhängig voneinander für einen gesättigten, geradkettigen oder verzweigten Kohlenwasserstoffrest mit 1 bis 4 Kohlenstoffatomen steht und

A für ein gegebenenfalls ein- oder mehrfach substituiertes Kohlenwasserstoffbrückengruppe (wie insbesondere ein gegebenenfalls ein- oder mehrfach substituiertes $C_1$-$C_7$-Alkylen oder $C_2$-$C_7$-Alkenylen) steht; wobei geeignete Substituenten z.B. ausgewählt sind unter OH, $NH_2$, $NO_2$, oder $C(O)OR_3$, insbesondere OH und $C(O)OR_3$, wobei $R_3$ wie oben definiert ist.

Besonders geeignet sind Verbindungen der Formel 1, worin

$R_1$ für einen $C_1$-, $C_2$- oder $C_3$-Alkylrest steht und
$R_2$ für einen substituierten Phenylrest steht, wobei der Substituent für HO- oder einen Esterrest der Formel $R_{1a}OC(O)$- steht der sich in para-, meta- oder insbesondere ortho-Stellung zum Rest $R_1OC(O)$- am aromatischen Ring befindet.

Als insbesondere geeignete Quaternisierungsmittel sind die Niedrigalkylester der Salicylsäure zu nennen sich, wie Methylsalicylat, Ethylsalicylat, n- und i-Propylsalicylat, und n-, i- oder tert-Butylsalicylat.

Oben genannte Ester werden typischerweise in Gegenwart von Säuren, insbesondere in Gegenwart von freien Protonensäuren, wie vor allem mit $C_{1-12}$-Monocarbonsäuren, wie Ameisensäure, Essigsäure oder Propionsäure oder $C_{2-12}$-Dicarbonsäuren wie Oxalsäure oder Adipinsäure; oder auch in Gegenwart von Sulfonsäuren, wie Benzolsulfonsäure oder Toluolsulfonsäure oder wässrigen Mineralsäuren, wie Schwefelsäure oder Salzsäure, eingesetzt.

c) in einer weiteren besonderen Ausführungsform erfolgt die Quaternisierung des mindestens einen quaternisierbaren tertiären Stickstoffatoms mit mindestens einem Quaternisierungsmittel ausgewählt aus Epoxiden, insbesondere Kohlenwasserstoff-Epoxiden.

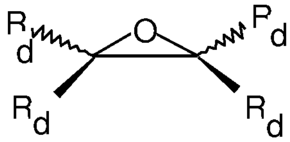

(4)

wobei die darin enthaltenen $R_d$ Reste gleich oder verschieden sind und für H oder für einen aliphatischen oder aromatischen Rest mit wenigstens 1 bis 10 Kohlenstoffatomen stehen, wobei die aliphatischen Reste $R_d$ zusätzlich Heteroatome, wie O, N, NH, S, in ihrer Kette enthalten können;
Insbesondere sind dies aliphatische oder aromatische Reste, wie z.B. lineare oder verzweigte $C_{1-10}$-Alkylreste oder aromatische Reste, wie Phenyl oder $C_{1-4}$-Alkylphenyl.

Als Kohlenwasserstoff-Epoxide eignen sich beispielsweise aliphatische und aromatische Alkylenoxide, wie insbesondere $C_{2-12}$-Alkylenoxide, wie Ethylenoxid, Propylenoxid, 1,2-Butylenoxid, 2,3-Butylenoxid, 2-Methyl-1,2-propenoxid (Isobutenoxid), 1,2-Pentenoxid, 2,3-Pentenoxid, 2-Methyl-1,2-butenoxid, 3-Methyl-1,2-butenoxid, 1,2-Hexenoxid, 2,3-Hexenoxid, 3,4-Hexenoxid, 2-Methyl-1,2-pentenoxid, 2-Ethyl-1,2-butenoxid, 3-Methyl-1,2-pentenoxid, 1,2-Decenoxid, 1,2-Dodecenoxid oder 4-Methyl-1,2-pentenoxid; sowie aromatensubstituierte Ethylenoxide, wie gegebenenfalls substituiertes Styroloxid, insbesondere Styroloxid oder 4-Methyl-styroloxid.

Im Falle der Verwendung von Epoxiden als Quaternisierungsmittel werden diese in Gegenwart oder in Abwesenheit von freien Säuren, insbesondere in Gegenwart oder Abwesenheit von freien Protonensäuren, wie vor allem mit $C_{1-12}$-Monocarbonsäuren, wie Ameisensäure, Essigsäure oder Propionsäure oder $C_{2-12}$-Dicarbonsäuren wie Oxalsäure oder Adipinsäure; oder auch in Gegenwart oder Abwesenheit von Sulfonsäuren, wie Benzolsulfonsäure oder Toluolsulfonsäure oder wässrigen Mineralsäuren, wie Schwefelsäure oder Salzsäure, eingesetzt. Das so hergestellte Quaternisierungsprodukt ist damit entweder " säurehaltig" oder " säurefrei" im Sinne der vorliegenden Erfindung.

**A5) Herstellung erfindungsgemäßer Additive:**

a) Quaternisierung

Die Quaternisierung wird in an sich bekannter Weise durchgeführt

(1) Zur Durchführung der Quaternisierung versetzt man das tertiäre Amin mit wenigstens einer Verbindung obiger Formel 1 oder 2, insbesondere in den erforderlichen stöchiometrischen Mengen, um die gewünschte Quaternisierung zu erreichen. Pro Äquivalent an quaternisierbarem tertiären Stickstoffatom kann man z.B. 0,1 bis 5,0, oder 0,2 bis 3,0, oder 0,5 bis 2,5 Äquivalente, an Quaternisierungsmittel einsetzen. Insbesondere werden aber etwa 1 bis 2 Äquivalente Quaternisierungsmittel in Relation zum tertiären Amin eingesetzt, um die tertiäre Amingruppe vollständig zu quaternisieren Man arbeitet hierbei typischerweise bei Temperaturen im Bereich von 50 bis 180°C, wie z.B. 90 bis 160 °C oder 100 bis 140 °C. Die Reaktionsdauer kann dabei im Bereich von wenigen Minuten oder einigen Stunden, wie z.B. etwa 10 Minuten bis zu etwa 24 Stunden liegen. Der Umsetzung kann dabei bei etwa 0,1 bis 20 bar, wie z.B. 1 bis 10 oder 1,5 bis 3 bar Druck, insbesondere aber etwa bei Normaldruck erfolgen.

Falls erforderlich können die Reaktanden in einem geeigneten inerten organischen aliphatischen oder aromatischen Lösungsmittel oder einem Gemisch davon, für die Quaternisierung vorgelegt werden. Typischen Beispiele sind z.B. Lösungsmittel der Solvesso Serie, Toluol oder Xylol oder Ethylhexanol Die Quaternisierung kann aber auch in Abwesenheit eines Lösungsmittels durchgeführt werden

Zur Durchführung der Quaternisierung kann die Zugabe katalytisch wirksamer Mengen einer Säure zweckmäßig sein. Bevorzugt sind dabei aliphatische Monocarbonsäuren, wie z.B. $C_1$-$C_{18}$-Monocarbonsäuren wie insbesondere Laurinsäure, Isononansäure oder 3,3,5, Trimethylhexansäure oder Neodecansäure, aber auch aliphatische Dicarbonsäuren oder höherwertige aliphatische Carbonsäuren mit einer C-Atomzahl im oben angegebenen Bereich. Die Quaternisierung kann auch in Anwesenheit einer Lewis-Säure durchgeführt werden. Die Quaternisierung kann aber auch in Abwesenheit

jeglicher Säure durchgeführt werden.

(2) Die Quaternierung mit einem Epoxid der Formel (4) erfolgt ebenfalls in an sich bekannter Weise. Liegt die Siedetemperatur einer Komponente des Reaktionsgemisches, insbesondere des Epoxides, bei Normaldruck oberhalb der Reaktionstemperatur, wird die Reaktion zweckmäßigerweise in einem Autoklaven durchgeführt.

Beispielsweise wird in einem Autoklaven eine Lösung des tertiären Amins mit der organischen Säure (wie z.B. Essigsäure) in den erforderlichen stöchiometrischen Mengen versetzt. Pro Äquivalent an quaternisierbarem tertiären Stickstoffatom kann man z.B. 0,1 bis 2,0, 0,2 bis 1,5, oder 0,5 bis 1,25 Äquivalente an Säure einsetzen. Insbesondere werden aber etwa annähernd äquimolare Anteile der Säure eingesetzt. Anschließend wird ausreichend mit $N_2$ gespült, sowie ein geeigneter Vordruck eingestellt und das Epoxid (z.B. Propylenoxid) wird in den erforderlichen stöchiometrischen Mengen bei einer Temperatur zwischen 20°C und 180°C zudosiert. Pro Äquivalent an quaternisierbarem tertiären Stickstoffatom kann man z.B. 0,1 bis 4,0 , 0,2 bis 3, oder 0,5 bis 2 Äquivalente an Epoxid einsetzen. Insbesondere werden aber etwa 1 bis 2 Äquivalente Epoxid in Relation zum tertiären Amin eingesetzt, um die tertiäre Amingruppe vollständig zu quaternisieren. Anschließend wird über eine geeignet langen Zeitraum von wenigen Minuten bis etwa 24 Stunden, wie z.B. etwa 10 h bei einer Temperatur zwischen 20°C und 180°C (z.B. 50°C) nachgerührt, abgekühlt, wie z.B. auf etwa 20 bis 50°C, mit $N_2$ gespült und der Reaktor entleert.

Die Umsetzung kann dabei bei etwa 0,1 bis 20 bar, wie z.B. 1 bis 10 oder 1,5 bis 5 bar Druck erfolgen. Die Umsetzung kann aber auch bei Normaldruck erfolgen. Insbesondere ist eine Inertgas-Atmosphäre, wie z.B. Stickstoff, zweckmäßig.

Falls erforderlich können die Reaktanden in einem geeigneten inerten organischen aliphatischen oder aromatischen Lösungsmittel oder einem Gemisch davon, für die Quaternisierung vorgelegt werden. Typische Beispiele sind z.B. Lösungsmittel der Solvesso Serie, Toluol oder Xylol oder 2-Ethylhexanol. Die Quaternisierung kann aber auch in Abwesenheit eines Lösungsmittels durchgeführt werden.

Die Quaternisierung kann in Gegenwart eines protischen Lösungsmittels durchgeführt werden, gegebenenfalls auch in Kombination mit einem aliphatischen oder aromatischen Lösungsmittel. Geeignete protische Lösungsmittel haben insbesondere eine Dielktrizitätskonstante (bei 20°C) von größer 7. Das protische Lösungsmittel kann eine oder mehrere OH-Gruppen enthalten und kann auch Wasser sein. Geeignete Lösungsmittel können auch Alkohole, Glykole und Glykolether sein. Insbesondere können geeignete protische Lösungsmittel solche sein, die in WO 2010132259 genannt sind. Insbesondere geeignete Lösungsmittel sind Methanol, Ethanol, n-Propanol, isoPropanol, alle Isomeren des Butanols, alle Isomeren des Pentanols, alle Isomeren des Hexanols, 2-Ethylhexanol, 2-Propylheptanol, sowie auch Gemische verschiedener Alkohole. Die Anwesenheit eines protischen Lösungsmittels kann den Umsatz und die Reaktionsgeschwindigkeit der Quaternierung positiv beeinflussen.

b) Aufarbeitung des Reaktionsgemisches

Das so gebildete Reaktionsendprodukt kann theoretisch weiter aufgereinigt oder das Lösungsmittel kann entfernt werden. Gegebenenfalls kann überschüssiges Reagenz, wie beispielsweise überschüssiges Epoxid, entfernt werden. Dies kann beispielsweise durch Einleiten von Stickstoff bei Normaldruck oder unter vermindertem Druck geschehen. Um die weitere Prozessierbarkeit der Produkte zu verbessern kann aber auch nach der Reaktion Lösungsmittel zugesetzt werden, wie z.B. Lösungsmittel der Solvesso Reihe, 2-Ethylhexanol, oder im Wesentlichen aliphatische Lösungsmittel. Gewöhnlich ist dies aber nicht zwingend notwendig, so dass das Reaktionsprodukt ohne weitere Aufreinigung als Additiv, gegebenenfalls nach Abmischung mit weiteren Additivkomponenten (s. unten) einsetzbar ist.

**B) Weitere Additivkomponenten**

Der mit dem erfindungsgemäßen quaternisierten Additiv additivierte Kraftstoff ist ein Ottokraftstoff oder insbesondere ein Mitteldestillat-Kraftstoff, vor allem ein Dieselkraftstoff.

Der Kraftstoff kann weitere übliche Additive zur Wirksamkeitsverbesserung und/oder Verschleißunterdrückung enthalten.

Im Falle von Dieselkraftstoffen sind dies in erster Linie übliche Detergenz-Additive, Trägeröle, Kaltfließverbesserer, Schmierfähigkeitsverbesserer (Lubricity Improver), Korrosionsinhibitoren, Demulgatoren, Dehazer, Antischaummittel, Cetanzahlverbesserer, Verbrennungsverbesserer, Antioxidantien oder Stabilisatoren, Antistatika, Metallocene, Metalldeaktivatoren, Farbstoffe und/oder Lösungsmittel.

Im Falle von Ottokraftstoffen sind dies vor allem Schmierfähigkeitsverbesserer (Friction Modifier), Korrosionsinhibitoren, Demulgatoren, Dehazer, Antischaummittel, Verbrennungsverbesserer, Antioxidantien oder Stabilisatoren, Antistatika, Metallocene, Metalldeaktivatoren, Farbstoffe und/oder Lösungsmittel.

Typische Beispiele geeigneter Co-Additive sind im folgenden Abschnitt aufgeführt:

B1) Detergenz-Additive

Vorzugsweise handelt es sich bei den üblichen Detergenz-Additiven um amphiphile Substanzen, die mindestens einen hydrophoben Kohlenwasserstoffrest mit einem zahlengemittelten Molekulargewicht ($M_n$) von 85 bis 20.000 und mindestens eine polare Gruppierung besitzen, die ausgewählt ist unter:

- (Da) Mono- oder Polyaminogruppen mit bis zu 6 Stickstoffatomen, wobei mindestens ein Stickstoffatom basische Eigenschaften hat;
- (Db) Nitrogruppen, gegebenenfalls in Kombination mit Hydroxylgruppen;
- (Dc) Hydroxylgruppen in Kombination mit Mono- oder Polyaminogruppen, wobei mindestens ein Stickstoffatom basische Eigenschaften hat;
- (Dd) Carboxylgruppen oder deren Alkalimetall- oder Erdalkalimetallsalzen;
- (De) Sulfonsäuregruppen oder deren Alkalimetall- oder Erdalkalimetallsalzen;
- (Df) Polyoxy-$C_2$- bis $C_4$-alkylengruppierungen, die durch Hydroxylgruppen, Mono- oder Polyaminogruppen, wobei mindestens ein Stickstoffatom basische Eigenschaften hat, oder durch Carbamatgruppen terminiert sind;
- (Dg) Carbonsäureestergruppen;
- (Dh) aus Bernsteinsäureanhydrid abgeleiteten Gruppierungen mit Hydroxy- und/oder Amino- und/oder Amido- und/oder Imidogruppen; und/oder
- (Di) durch Mannich-Umsetzung von substituierten Phenolen mit Aldehyden und Mono- oder Polyaminen erzeugten Gruppierungen.

Der hydrophobe Kohlenwasserstoffrest in den obigen Detergenz-Additiven, welcher für die ausreichende Löslichkeit im Kraftstoff sorgt, hat ein zahlengemitteltes Molekulargewicht ($M_n$) von 85 bis 20.000, vorzugsweise von 113 bis 10.000, besonders bevorzugt von 300 bis 5.000, stärker bevorzugt von 300 bis 3.000, noch stärker bevorzugt von 500 bis 2.500 und insbesondere von 700 bis 2.500, vor allem von 800 bis 1500. Als typischer hydrophober Kohlenwasserstoffrest, insbesondere in Verbindung mit den polaren insbesondere Polypropenyl-, Polybutenyl- und Polyisobutenylreste mit einem zahlenmittleren Molekulargewicht $M_n$ von vorzugsweise jeweils 300 bis 5.000, besonders bevorzugt 300 bis 3.000, stärker bevorzugt 500 bis 2.500 noch stärker bevorzugt 700 bis 2.500 und insbesondere 800 bis 1.500 in Betracht.

Als Beispiele für obige Gruppen von Detergenz-Additiven seien die folgenden genannt:

Mono- oder Polyaminogruppen (Da) enthaltende Additive sind vorzugsweise Polyalkenmono- oder Polyalkenpolyamine auf Basis von Polypropen oder von hochreaktivem (d.h. mit überwiegend endständigen Doppelbindungen) oder konventionellem (d.h. mit überwiegend mittenständigen Doppelbindungen) Polybuten oder Polyisobuten mit $M_n$ = 300 bis 5000, besonders bevorzugt 500 bis 2500 und insbesondere 700 bis 2500. Derartige Additive auf Basis von hochreaktivem Polyisobuten, welche aus dem Polyisobuten, das bis zu 20 Gew.-% n-Buten-Einheiten enthalten kann, durch Hydroformylierung und reduktive Aminierung mit Ammoniak, Monoaminen oder Polyaminen wie Dimethylaminopropylamin, Ethylendiamin, Diethylentriamin, Triethylentetramin oder Tetraethylenpentamin hergestellt werden können, sind insbesondere aus der EP-A 244 616 bekannt. Geht man bei der Herstellung der Additive von Polybuten oder Polyisobuten mit überwiegend mittenständigen Doppelbindungen (meist in der β- und γ-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier Amine, wie z. B. Ammoniak, Monoamine oder die oben genannten Polyamine, eingesetzt werden. Entsprechende Additive auf Basis von Polypropen sind insbesondere in der WO-A 94/24231 beschrieben.

Weitere besondere Monoaminogruppen (Da) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in der WO-A 97/03946 beschrieben sind.

Weitere besondere Monoaminogruppen (Da) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in der DE-A 196 20 262 beschrieben sind.

Nitrogruppen (Db), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in der WO-A96/03367 und in der WO-A 96/03479 beschrieben sind. Diese Umsetzungsprodukte stellen in der Regel Mischungen aus reinen Nitropolyisobutenen (z. B. α ,β -Dinitropolyisobuten) und gemischten Hydroxynitropolyisobutenen (z. B. α -Nitro-β -hydroxypolyisobuten) dar.

Hydroxylgruppen in Kombination mit Mono- oder Polyaminogruppen (Dc) enthaltende Additive sind insbeson-

dere Umsetzungsprodukte von Polyisobutenepoxiden, erhältlich aus vorzugsweise überwiegend endständige Doppelbindungen aufweisendem Polyisobuten mit $M_n$ = 300 bis 5000 mit Ammoniak, Mono- oder Polyaminen, wie sie insbesondere in der EP-A 476 485 beschrieben sind.

Carboxylgruppen oder deren Alkalimetall- oder Erdalkalimetallsalze (Dd) enthaltende Additive sind vorzugsweise Copolymere von $C_2$- bis $C_{40}$-Olefinen mit Maleinsäureanhydrid mit einer Gesamt-Molmasse von 500 bis 20.000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A 307 815 bekannt. Derartige Additive dienen hauptsächlich zur Verhinderung von Ventilsitzverschleiß und können, wie in der WO-A 87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (De) enthaltende Additive sind vorzugsweise Alkalimetall- oder Erdalkalimetallsalze eines Sulfobernsteinsäurealkylesters, wie er insbesondere in der EP-A 639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)buten-aminen oder Polyetheraminen eingesetzt werden.

Polyoxy-$C_2$-$C_4$-alkylengruppierungen (Df) enthaltende Additive sind vorzugsweise Polyether oder Polyetheramine, welche durch Umsetzung von $C_2$- bis $C_{60}$-Alkanolen, $C_6$- bis $C_{30}$-Alkandiolen, Mono- oder Di-$C_2$- bis $C_{30}$-alkylaminen, $C_1$- bis $C_{30}$-Alkylcyclo-hexanolen oder $C_1$- bis $C_{30}$-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und US-A 4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (Dg) enthaltende Additive sind vorzugsweise Ester aus Mono-, Di- oder Tricarbonsäuren mit langkettigen Alkanolen oder Polyolen, insbesondere solche mit einer Mindestviskosität von 2 $mm^2/s$ bei 100 °C, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder insbesondere Imidogruppen (Dh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit $M_n$ = vorzugsweise 300 bis 5000, besonders bevorzugt 300 bis 3000, stärker bevorzugt 500 bis 2500, noch stärker bevorzugt 700 bis 2500 und insbesondere 800 bis 1500, mit Maleinsäureanhydrid auf thermischem Weg in einer En-Reaktion oder über das chlorierte Polyisobuten erhältlich sind. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Beim Vorliegen von Imidogruppierungen D(h) wird das weitere Detergenz-Additiv im Sinne der vorliegenden Erfindung jedoch nur bis maximal 100 % der Gewichtsmenge an Verbindungen mit Betainstruktur eingesetzt. Derartige Kraftstoffadditive sind allgemein bekannt und beispielsweise in den Dokumenten (1) und (2) beschrieben. Bevorzugt handelt es sich um die Umsetzungsprodukte von Alkyl- oder Alkenyl-substituierten Bernsteinsäuren oder Derivaten davon mit Aminen und besonders bevorzugt um die Umsetzungsprodukte von Polyisobutenyl-substituierten Bernsteinsäuren oder Derivaten davon mit Aminen. Von besonderem Interesse sind hierbei Umsetzungsprodukte mit aliphatischen Polyaminen (Polyalkylenimine) wie insbesondere Ethylendiamin, Diethylentriamin, Triethylentetramin, Tetraethylenpentamin, Pentaethylenhexamin und Hexaethylenheptamin, welche eine Imidstruktur aufweisen.

Durch Mannich-Umsetzung von substituierten Phenolen mit Aldehyden und Mono- oder Polyaminen erzeugte Gruppierungen (Di) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit $M_n$ = 300 bis 5000 stammen. Derartige "Polyisobuten-Mannichbasen" sind insbesondere in der EP-A 831 141 beschrieben.

Dem Kraftstoff können ein oder mehrere der genannten Detergenz-Additive in solch einer Menge zugegeben

werden, dass die Dosierrate an diesen Detergenz-Additiven vozugsweise 25 bis 2500 Gew.-ppm, insbesondere 75 bis 1500 Gew.-ppm, vor allem 150 bis 1000 Gew.-ppm, beträgt.

B2) Trägeröle

Mitverwendete Trägeröle können mineralischer oder synthetischer Natur sein. Geeignete mineralische Trägeröle sind bei der Erdölverarbeitung anfallende Fraktionen, wie Brightstock oder Grundöle mit Viskositäten wie beispielsweise aus der Klasse SN 500 bis 2000, aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500 °C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind Polyolefine (Polyalphaolefine oder Polyinternalolefine), (Poly)ester, Poly)alkoxylate, Polyether, aliphatische Polyetheramine, alkylphenolgestartete Polyether, alkylphenolgestartete Polyetheramine und Carbonsäureester langkettiger Alkanole.

Beispiele für geeignete Polyolefine sind Olefinpolymerisate mit $M_n$ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-$C_2$-bis $C_4$-alkylengruppierungen enthaltende Verbindungen, welche durch Umsetzung von $C_2$- bis $C_{60}$-Alkanolen, $C_6$- bis $C_{30}$-Alkandiolen, Mono- oder Di-$C_2$- bis $C_{30}$-alkylaminen, $C_1$- bis $C_{30}$-Alkyl-cyclohexanolen oder $C_1$- bis $C_{30}$-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und der US-A 4,877,416 beschrieben. Beispielsweise können als Polyetheramine Poly-$C_2$- bis $C_6$-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 Kohlenstoffatomen. Typische Vertreter der Ester sind Adipate, Phthalate, iso-Phthalate, Terephthalate und Trimellitate des Isooctanols, Isononanols, Isodecanols und des Iso-tridecanols, z. B. Di-(n- oder Isotridecyl)phthalat.

Weitere geeignete Trägerölsysteme sind beispielsweise in der DE-A 38 26 608, DE-A 41 42 241, DE-A 43 09 074, EP-A 452 328 und der EP-A 548 617 beschrieben.

Beispiele für besonders geeignete synthetische Trägeröle sind alkoholgestartete Polyether mit etwa 5 bis 35, vorzugsweise etwa 5 bis 30, besonders bevorzugt 10 bis 30 und insbesondere 15 bis 30 $C_3$- bis $C_6$-Alkylenoxideinheiten, z. B. Propylenoxid-, n-Butylenoxid- und Isobutylenoxid-Einheiten oder Gemischen davon, pro Alkoholmolekül. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten $C_6$- bis $C_{18}$-Alkylrest steht. Als besondere Beispiele sind zu nennen Tridecanol und Nonylphenol. Besonders bevorzugte alkoholgestartete Polyether sind die Umsetzungsprodukte (Polyveretherungsprodukte) von einwertigen aliphatischen $C_6$- bis $C_{18}$-Alkoholen mit $C_3$- bis $C_6$-Alkylenoxiden. Beispiele für einwertige aliphatische $C_6$-$C_{18}$-Alkohole sind Hexanol, Heptanol, Octanol, 2-Ethylhexanol, Nonylalkohol, Decanol, 3-Propylheptanol, Undecanol, Dodecanol, Tridecanol, Tetradecanol, Pentadecanol, Hexadecanol, Octadecanol und deren Konstitutions- und Stellungsisomere. Die Alkohole können sowohl in Form der reinen Isomere als auch in Form technischer Gemische eingesetzt werden. Ein besonders bevorzugter Alkohol ist Tridecanol. Beispiele für $C_3$- bis $C_6$-Alkylenoxide sind Propylenoxid, wie 1,2-Propylenoxid, Butylenoxid, wie 1,2-Butylenoxid, 2,3-Butylenoxid, Isobutylenoxid oder Tetrahydrofuran, Pentylenoxid und Hexylenoxid. Besonders bevorzugt sind hierunter $C_3$- bis $C_4$-Alkylenoxide, d.h. Propylenoxid wie 1,2-Propylenoxid und Butylenoxid wie 1,2-Butylenoxid, 2,3-Butylenoxid und Isobutylenoxid. Speziell verwendet man Butylenoxid.

Weitere geeignete synthetische Trägeröle sind alkoxylierte Alkylphenole, wie sie in der DE-A 10 102 913 beschrieben sind.

Besondere Trägeröle sind synthetische Trägeröle, wobei die zuvor beschriebenen alkoholgestarteten Polyether besonders bevorzugt sind.

Das Trägeröl bzw. das Gemisch verschiedener Trägeröle wird dem Kraftstoff in einer Menge von vorzugsweise 1 bis 1000 Gew.-ppm, besonders bevorzugt von 10 bis 500 Gew.-ppm und insbesondere von 20 bis 100 Gew.-ppm zugesetzt.

B3) Kaltfließverbesserer

Geeignete Kaltfließverbesserer sind im Prinzip alle organischen Verbindungen, welche in der Lage sind, das Fließverhalten von Mitteldestillat-Kraftstoffen bzw. Dieselkraftstoffen in der Kälte zu verbessern. Zweckmäßigerweise müssen sie eine ausreichende Öllöslichkeit aufweisen. Insbesondere kommen hierfür die üblicherweise bei Mitteldestillaten aus fossilem Ursprung, also bei üblichen mineralischen Dieselkraftstoffen, eingesetzten Kaltfließverbesserer (" middle distillate flow improvers" , " MDFI" ) in Betracht. Jedoch können auch organische Verbindungen verwendet werden, die beim Einsatz in üblichen Dieselkraftstoffen zum Teil oder überwiegend die Eigenschaften eines Wax Anti-Settling Additivs (" WASA" ) aufweisen. Auch können sie zum Teil oder überwiegend als Nukleatoren wirken. Es können aber auch Mischungen aus als MDFI wirksamen und/oder als WASA wirksamen und/oder als Nukleatoren wirksamen organischen Verbindungen eingesetzt werden.

Typischerweise wird der Kaltfließverbesserer ausgewählt aus:

- (K1) Copolymeren eines $C_2$- bis $C_{40}$-Olefins mit wenigstens einem weiteren ethylenisch ungesättigten Monomer;
- (K2) Kammpolymeren;
- (K3) Polyoxyalkylenen;
- (K4) polaren Stickstoffverbindungen;
- (K5) Sulfocarbonsäuren oder Sulfonsäuren oder deren Derivaten; und
- (K6) Poly(meth)acrylsäureestern.

Es können sowohl Mischungen verschiedener Vertreter aus einer der jeweiligen Klassen (K1) bis (K6) als auch Mischungen von Vertretern aus verschiedenen Klassen (K1) bis (K6) eingesetzt werden.

Geeignete $C_2$- bis $C_{40}$-Olefin-Monomere für die Copolymeren der Klasse (K1) sind beispielsweise solche mit 2 bis 20, insbesondere 2 bis10 Kohlenstoffatomen sowie mit 1 bis 3, vorzugsweise mit 1 oder 2, insbesondere mit einer Kohlenstoff-Kohlenstoff-Doppelbindung. Im zuletzt genannten Fall kann die Kohlenstoff-Kohlenstoff-Doppelbindung sowohl terminal ($\alpha$ -Olefine) als auch intern angeordnet sein kann. Bevorzugt sind jedoch $\alpha$ -Olefine, besonders bevorzugt $\alpha$ -Olefine mit 2 bis 6 Kohlenstoffatomen, beispielsweise Propen, 1-Buten, 1-Penten, 1-Hexen und vor allem Ethylen.

Bei den Copolymeren der Klasse (K1) ist das wenigstens eine weitere ethylenisch ungesättigte Monomer vorzugsweise ausgewählt unter Carbonsäurealkenylestern, (Meth)Acrylsäureestern und weiteren Olefinen.

Werden weitere Olefine mit einpolymerisiert, sind dies vorzugsweise höhermolekulare als das oben genannte $C_2$- bis $C_{40}$-Olefin-Basismonomere. Setzt man beispielsweise als Olefin-Basismonomer Ethylen oder Propen ein, eignen sich als weitere Olefine insbesondere $C_{10}$- bis $C_{40}$-$\alpha$ -Olefine. Weitere Olefine werden in den meisten Fällen nur dann mit einpolymerisiert, wenn auch Monomere mit Carbonsäureester-Funktionen eingesetzt werden.

Geeignete (Meth)Acrylsäureester sind beispielsweise Ester der (Meth)Acrylsäure mit $C_1$- bis $C_{20}$-Alkanolen, insbesondere $C_1$- bis $C_{10}$-Alkanolen, vor allem mit Methanol, Ethanol, Propanol, Isopropanol, n-Butanol, sec.-Butanol, Isobutanol, tert.-Butanol, Pentanol, Hexanol, Heptanol, Octanol, 2-Ethylhexanol, Nonanol und Decanol sowie Strukturisomeren hiervon.

Geeignete Carbonsäurealkenylester sind beispielsweise $C_2$- bis $C_{14}$-Alkenylester, z.B. die Vinyl- und Propenylester, von Carbonsäuren mit 2 bis 21 Kohlenstoffatomen, deren Kohlenwasserstoffrest linear oder verzweigt sein kann. Bevorzugt sind hierunter die Vinylester. Unter den Carbonsäuren mit verzweigtem Kohlenwasserstoffrest sind solche bevorzugt, deren Verzweigung sich in der $\alpha$-Position zur Carboxylgruppe befindet, wobei das $\alpha$-Kohlenstoffatom besonders bevorzugt tertiär ist, d. h. die Carbonsäure eine sogenannte Neocarbonsäure ist. Vorzugsweise ist der Kohlenwasserstoffrest der Carbonsäure jedoch linear.

Beispiele für geeignete Carbonsäurealkenylester sind Vinylacetat, Vinylpropionat, Vinylbutyrat, Vinyl-2-ethylhexanoat, Neopentansäurevinylester, Hexansäurevinylester, Neononansäurevinylester, Neodecansäurevinylester und die entsprechenden Propenyl-ester, wobei die Vinylester bevorzugt sind. Ein besonders bevorzugter Carbonsäurealkenylester ist Vinylacetat; typische hieraus resultierende Copolymere der Gruppe (K1) sind die mit am häufigsten eingesetzten Ethylen-Vinylacetat-Copolymere (" EVA" ).

Besonders vorteilhaft einsetzbare Ethylen-Vinylacetat-Copolymere und ihre Herstellung sind in der WO 99/29748 beschrieben.

Als Copolymere der Klasse (K1) sind auch solche geeignet, die zwei oder mehrere voneinander verschiedene Carbonsäurealkenylester einpolymerisiert enthalten, wobei diese sich in der Alkenylfunktion und/oder in der Carbonsäuregruppe unterscheiden. Ebenfalls geeignet sind Copolymere, die neben dem/den Carbonsäurealkenylester(n) wenigstens ein Olefin und/oder wenigstens ein (Meth)Acrylsäureester einpolymerisiert enthalten.

Auch Terpolymere aus einem $C_2$- bis $C_{40}$-$\alpha$ -Olefin, einem $C_1$- bis $C_{20}$-Alkylester einer ethylenisch ungesättigten Monocarbonsäure mit 3 bis 15 Kohlenstoffatomen und einem $C_2$- bis $C_{14}$-Alkenylester einer gesättigten Monocarbonsäure mit 2 bis 21 Kohlenstoffatomen sind als Copolymere der Klasse (K1) geeignet. Derartige Terpolymere sind in der

WO 2005/054314 beschrieben. Ein typisches derartiges Terpolymer ist aus Ethylen, Acrylsäure-2-ethylhexylester und Vinylacetat aufgebaut.

Das wenigstens eine oder die weiteren ethylenisch ungesättigten Monomeren sind in den Copolymeren der Klasse (K1) in einer Menge von vorzugsweise 1 bis 50 Gew.-%, insbesondere von 10 bis 45 Gew.-% und vor allem von 20 bis 40 Gew.-%, bezogen auf das Gesamtcopolymer, einpolymerisiert. Der gewichtsmäßige Hauptanteil der Monomereinheiten in den Copolymeren der Klasse (K1) stammt somit in der Regel aus den $C_2$- bis $C_{40}$-Basis-Olefinen.

Die Copolymere der Klasse (K1) weisen vorzugsweise ein zahlenmittleres Molekulargewicht $M_n$ von 1000 bis 20.000, besonders bevorzugt von 1000 bis 10.000 und insbesondere von 1000 bis 8000 auf.

Typische Kammpolymere der Komponente (K2) sind beispielsweise durch die Copolymerisation von Maleinsäureanhydrid oder Fumarsäure mit einem anderen ethylenisch ungesättigten Monomer, beispielsweise mit einem $\alpha$ -Olefin oder einem ungesättigten Ester wie Vinylacetat, und anschließende Veresterung der Anhydrid- bzw. Säurefunktion mit einem Alkohol mit wenigstens 10 Kohlenstoffatomen erhältlich. Weitere geeignete Kammpolymere sind Copolymere von $\alpha$ -Olefinen und veresterten Comonomeren, beispielsweise veresterte Copolymere von Styrol und Maleinsäureanhydrid oder veresterte Copolymere von Styrol und Fumarsäure. Geeignete Kammpolymere können auch Polyfumarate oder Polymaleinate sein. Außerdem sind Homo- und Copolymere von Vinylethern geeignete Kammpolymere. Als Komponente der Klasse (K2) geeignete Kammpolymere sind beispielsweise auch solche, die in der WO 2004/035715 und in " Comb-Like Polymers. Structure and Properties" , N. A. Platé und V. P. Shibaev, J. Poly. Sci. Macromolecular Revs. 8, Seiten 117 bis 253 (1974)" beschrieben sind. Auch Gemische von Kammpolymeren sind geeignet.

Als Komponente der Klasse (K3) geeignete Polyoxyalkylene sind beispielsweise Polyoxyalkylenester, Polyoxyalkylenether, gemischte Polyoxyalkylenesterether und Gemische davon. Bevorzugt enthalten diese Polyoxyalkylenverbindungen wenigstens eine, vorzugsweise wenigstens zwei lineare Alkylgruppen mit jeweils 10 bis 30 Kohlenstoffatomen und eine Polyoxyalkylengruppe mit einem zahlenmittleren Molekulargewicht von bis zu 5000. Derartige Polyoxyalkylenverbindungen sind beispielsweise in der EP-A 061 895 sowie in der US 4 491 455 beschrieben. Besondere Polyoxyalkylenverbindungen basieren auf Polyethylenglykolen und Polypropylenglykolen mit einem zahlenmittleren Molekulargewicht von 100 bis 5000. Weiterhin sind Polyoxyalkylenmono- und -diester von Fettsäuren mit 10 bis 30 Kohlenstoffatomen wie Stearinsäure oder Behensäure geeignet.

Als Komponente der Klasse (K4) geeignete polare Stickstoffverbindungen können sowohl ionischer als auch nicht ionischer Natur sein und besitzen vorzugsweise wenigstens einen, insbesondere wenigstens zwei Substituenten in Form eines tertiären Stickstoffatoms der allgemeinen Formel $>NR^7$, worin $R^7$ für einen Cs- bis $C_{40}$-Kohlenwasserstoffrest steht. Die Stickstoffsubstituenten können auch quaternisiert, das heißt in kationischer Form, vorliegen. Beispiele für solche Stickstoffverbindungen sind Ammoniumsalze und/oder Amide, die durch die Umsetzung wenigstens eines mit wenigstens einem Kohlenwasserstoffrest substituierten Amins mit einer Carbonsäure mit 1 bis 4 Carboxylgruppen bzw. mit einem geeignetem Derivat davon erhältlich sind. Vorzugsweise enthalten die Amine wenigstens einen linearen $C_8$- bis $C_{40}$-Alkylrest. Zur Herstellung der genannten polaren Stickstoffverbindungen geeignete primäre Amine sind beispielsweise Octylamin, Nonylamin, Decylamin, Undecylamin, Dodecylamin, Tetradecylamin und die höheren linearen Homologen, hierzu geeignete sekundäre Amine sind beispielsweise Dioctadecylamin und Methylbehenylamin. Geeignet sind hierzu auch Amingemische, insbesondere großtechnisch zugängliche Amingemische wie Fettamine oder hydrierte Tallamine, wie sie beispielsweise in Ullmanns Encyclopedia of Industrial Chemistry, 6. Auflage, im Kapitel " Amines, aliphatic" beschrieben werden. Für die Umsetzung geeignete Säuren sind beispielsweise Cyclohexan-1,2-dicarbonsäure, Cyclohexen-1,2-dicarbonsäure, Cyclopentan-1,2-dicarbonsäure, Naphthalindicarbonsäure, Phthalsäure, Isophthalsäure, Terephthalsäure und mit langkettigen Kohlenwasserstoffresten substituierte Bernsteinsäuren.

Insbesondere ist die Komponente der Klasse (K4) ein öllösliches Umsetzungsprodukt aus mindestens eine tertiäre Aminogruppe aufweisenden Poly($C_2$- bis $C_{20}$-Carbon-säuren) mit primären oder sekundären Aminen. Die diesem Umsetzungsprodukt zugrundeliegenden mindestens eine tertiäre Aminogruppe aufweisenden Poly($C_2$- bis $C_{20}$-Carbonsäuren) enthalten vorzugsweise mindestens 3 Carboxylgruppen, insbesondere 3 bis 12, vor allem 3 bis 5 Carboxylgruppen. Die Carbonsäure-Einheiten in den Polycarbonsäuren weisen vorzugsweise 2 bis 10 Kohlenstoffatome auf, insbesondere sind es Essigsäure-Einheiten. Die Carbonsäure-Einheiten sind in geeigneter Weise zu den Polycarbonsäuren verknüpft, meist über ein oder mehrere Kohlenstoff- und/oder Stickstoffatome. Vorzugsweise sind sie an tertiäre Stickstoffatome angebunden, die im Falle mehrerer Stickstoffatome über Kohlenwasserstoffketten verbunden sind.

Vorzugsweise ist die Komponente der Klasse (K4) ein öllösliches Umsetzungsprodukt auf Basis von mindestens eine tertiäre Aminogruppe aufweisenden Poly($C_2$- bis $C_{20}$-Carbonsäuren) der allgemeinen Formel IIa oder IIb

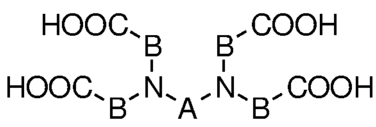

(IIa)

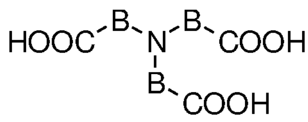

(IIb)

in denen die Variable A eine geradkettige oder verzweigte $C_2$- bis $C_6$-Alkylengruppe oder die Gruppierung der Formel III

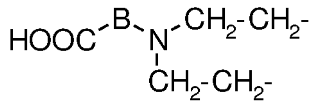

(III)

darstellt und die Variable B eine $C_1$- bis $C_{19}$-Alkylengruppe bezeichnet. Die Verbindungen der allgemeinen Formel IIa und IIb weisen insbesondere die Eigenschaften eines WASA auf.

Weiterhin ist das bevorzugte öllösliche Umsetzungsprodukt der Komponente (K4), insbesondere das der allgemeinen Formel IIa oder IIb, ein Amid, ein Amidammoniumsalz oder ein Ammoniumsalz, in dem keine, eine oder mehrere Carbonsäuregruppen in Amidgruppen übergeführt sind.

Geradkettige oder verzweigte $C_2$- bis $C_6$-Alkylengruppen der Variablen A sind beispielsweise 1,1-Ethylen, 1,2-Propylen, 1,3-Propylen, 1,2-Butylen, 1,3-Butylen, 1,4-Butylen, 2-Methyl-1,3-propylen, 1,5-Pentylen, 2-Methyl-1,4-butylen, 2,2-Dimethyl-1,3-propylen, 1,6-Hexylen (Hexamethylen) und insbesondere 1,2-Ethylen. Vorzugsweise umfasst die Variable A 2 bis 4, insbesondere 2 oder 3 Kohlenstoffatome.

$C_1$- bis $C_{19}$-Alkylengruppen der Variablen B sind vor beispielsweise 1,2-Ethylen, 1,3-Propylen, 1,4-Butylen, Hexamethylen, Octamethylen, Decamethylen, Dodecamethylen, Tetradecamethylen, Hexadecamethylen, Octadecamethylen, Nonadecamethylen und insbesondere Methylen. Vorzugsweise umfasst die Variable B 1 bis 10, insbesondere 1 bis 4 Kohlenstoffatome.

Die primären und sekundären Amine als Umsetzungspartner für die Polycarbonsäuren zur Bildung der Komponente (K4) sind üblicherweise Monoamine, insbesondere aliphatische Monoamine. Diese primären und sekundären Amine können aus einer Vielzahl von Aminen ausgewählt sein, die - gegebenenfalls miteinander verbundene - Kohlenwasserstoffreste tragen.

Meist sind diese den öllöslichen Umsetzungsprodukten der Komponente (K4) zugrundeliegenden Amine sekundären Amine und weisen die allgemeine Formel $HN(R^8)_2$ auf, in der die beiden Variablen $R^8$ unabhängig voneinander jeweils geradkettige oder verzweigte $C_{10}$- bis $C_{30}$-Alkylreste, insbesondere $C_{14}$- bis $C_{24}$-Alkylreste bedeuten. Diese längerkettigen Alkylreste sind vorzugsweise geradkettig oder nur in geringem Grade verzweigt. In der Regel leiten sich die genannten sekundären Amine hinsichtlich ihrer längerkettigen Alkylreste von natürlich vorkommenden Fettsäuren bzw. von deren Derivaten ab. Vorzugsweise sind die beiden Reste $R^8$ gleich.

Die genannten sekundären Amine können mittels Amidstrukturen oder in Form der Ammoniumsalze an die Polycarbonsäuren gebunden sein, auch kann nur ein Teil als Amidstrukturen und ein anderer Teil als Ammoniumsalze vorliegen. Vorzugsweise liegen nur wenige oder keine freien Säuregruppen vor. Vorzugsweise liegen die öllöslichen Umsetzungsprodukte der Komponente (K4) vollständig in Form der Amidstrukturen vor.

Typische Beispiele für derartige Komponenten (K4) sind Umsetzungsprodukte der Nitrilotriessigsäure, der Ethylendiamintetraessigsäure oder der Propylen-1,2-diamintetraessigsäure mit jeweils 0,5 bis 1,5 Mol pro Carboxylgruppe, insbesondere 0,8 bis 1,2 Mol pro Carboxylgruppe, Dioleylamin, Dipalmitinamin, Dikokosfettamin, Distearylamin, Dibehenylamin oder insbesondere Ditalgfettamin. Eine besonders bevorzugte Komponente (K4) ist das Umsetzungsprodukt aus 1 Mol Ethylendiamintetraessigsäure und 4 Mol hydriertem Ditalgfettamin.

Als weitere typische Beispiele für die Komponente (K4) seien die N,N-Dialkylammoniumsalze von 2-N' ,N' -Dialkylamidobenzoaten, beispielsweise das Reaktionsprodukt aus 1 Mol Phthalsäureanhydrid und 2 Mol Ditalgfettamin, wobei letzteres hydriert oder nicht hydriert sein kann, und das Reaktionsprodukt von 1 Mol eines Alkenylspirobislactons mit 2 Mol eines Dialkylamins, beispielsweise Ditalgfettamin und/oder Talgfettamin, wobei die beiden letzteren hydriert oder nicht hydriert sein können, genannt.

Weitere typische Strukturtypen für die Komponente der Klasse (K4) sind cyclische Verbindungen mit tertiären Aminogruppen oder Kondensate langkettiger primärer oder sekundärer Amine mit carbonsäurehaltigen Polymeren, wie sie in der WO 93/18115 beschrieben sind.

Als Kaltfließverbesserer der Komponente der Klasse (K5) geeignete Sulfocarbonsäuren, Sulfonsäuren oder deren Derivate sind beispielsweise die öllöslichen Carbonsäureamide und Carbonsäureester von ortho-Sulfobenzoesäure, in denen die Sulfonsäurefunktion als Sulfonat mit alkylsubstituierten Ammoniumkationen vorliegt, wie sie in der EP-A 261 957 beschrieben werden.

Als Kaltfließverbesserer der Komponente der Klasse (K6) geeignete Poly(meth)acrylsäureester sind sowohl Homo- als auch Copolymere von Acryl- und Methacrylsäureestern. Bevorzugt sind Copolymere von wenigstens zwei voneinander verschiedenen (Meth)Acrylsäureestern, die sich bezüglich des einkondensierten Alkohols unterscheiden. Gegebenenfalls enthält das Copolymer noch ein weiteres, davon verschiedenes olefinisch ungesättigtes Monomer einpolymerisiert. Das gewichtsmittlere Molekulargewicht des Polymers beträgt vorzugsweise 50.000 bis 500.000. Ein besonders bevorzugtes Polymer ist ein Copolymer von Methacrylsäure und Methacrylsäureestern von gesättigten $C_{14}$- und $C_{15}$-Alkoholen, wobei die Säuregruppen mit hydriertem Tallamin neutralisiert sind. Geeignete Poly(meth)acrylsäureester sind beispielsweise in der WO 00/44857 beschrieben.

Dem Mitteldestillat-Kraftstoff bzw. Dieselkraftstoff wird der Kaltfließverbesserer bzw. das Gemisch verschiedener Kaltfließverbesserer in einer Gesamtmenge von vorzugsweise 10 bis 5000 Gew.-ppm, besonders bevorzugt von 20 bis 2000 Gew.-ppm, stärker bevorzugt von 50 bis 1000 Gew.-ppm und insbesondere von 100 bis 700 Gew.-ppm, z.B. von 200 bis 500 Gew.-ppm, zugegeben.

B4) Schmierfähigkeitsverbesserer

Geeignete Schmierfähigkeitsverbesserer (Lubricity Improver bzw. Friction Modifier) basieren üblicherweise auf Fettsäuren oder Fettsäureestern. Typische Beispiele sind Tallölfettsäure, wie beispielsweise in der WO 98/004656 beschrieben, und Glycerinmonooleat. Auch die in der US 6 743 266 B2 beschriebenen Reaktionsprodukte aus natürlichen oder synthetischen Ölen, beispielsweise Triglyceriden, und Alkanolaminen sind als solche Schmierfähigkeitsverbesserer geeignet.

B5) Korrosionsinhibitoren

Geeignete Korrosionsinhibitoren sind z.B. Bernsteinsäureester, vor allem mit Polyolen, Fettsäurederivate, z.B. Ölsäureester, oligomerisierte Fettsäuren, substituierte Ethanolamine und Produkte, die unter dem Handelsnamen RC 4801 (Rhein Chemie Mannheim, Deutschland) oder HiTEC 536 (Ethyl Corporation) vertrieben werden.

B6) Demulgatoren

Geeignete Demulgatoren sind z.B. die Alkali- oder Erdalkalisalze von Alkyl-substituierten Phenol- und Naphthalinsulfonaten und die Alkali- oder Erdalkalisalze von Fettsäuren, außerdem neutrale Verbindungen wie Alkoholalkoxylate, z.B. Alkoholethoxylate, Phenolalkoxylate, z.B. tert-Butylphenolethoxylat oder tert-Pentylphenolethoxylat, Fettsäuren, Alkylphenole, Kondensationsprodunkte von Ethylenoxid (EO) und Propylenoxid (PO), z.B. auch in Form von EO/PO-Blockcopolymeren, Polyethylenimine oder auch Polysiloxane.

B7) Dehazer

Geeignete Dehazer sind z.B. alkoxylierte Phenol-Formaldehyd-Kondensate, wie beispielsweise die unter dem Handelsnamen erhältlichen Produkte NALCO 7D07 (Nalco) und TOLAD 2683 (Petrolite).

B8) Antischaummittel

Geeignete Antischaummittel sind z.B. Polyether-modifizierte Polysiloxane, wie beispielsweise die unter dem Handelsnamen erhältlichen Produkte TEGOPREN 5851 (Goldschmidt), Q 25907 (Dow Corning) und RHODOSIL (Rhone Poulenc).

B9) Cetanzahlverbesserer

Geeignete Cetanzahlverbesserer sind z.B. aliphatische Nitrate wie 2-Ethylhexylnitrat und Cyclohexylnitrat sowie Peroxide wie Di-tert-butylperoxid.

B10) Antioxidantien

Geeignete Antioxidantien sind z.B. substituierte Phenole, wie 2,6-Di-tert.-butylphenol und 6-Di-tert.-butyl-3-methylphenol sowie Phenylendiamine wie N,N'-Di-sec.-butyl-p-phenylendiamin.

B11) Metalldeaktivatoren

Geeignete Metalldeaktivatoren sind z.B. Salicylsäurederivate wie N,N'-Disalicyliden-1,2-propandiamin.

B12) Lösungsmittel

Geeignete sind z.B. unpolare organische Lösungsmittel wie aromatische und aliphatische Kohlenwasserstoffe, beispielsweise Toluol, Xylole, "white spirit" und Produkte, die unter dem Handelsnamen SHELLSOL (Royal Dutch/Shell Group) und EXXSOL (ExxonMobil) vertrieben werden, sowie polare organische Lösungsmittel, beispielsweise Alkohole wie 2-Ethylhexanol, Decanol und Isotridecanol. Derartige Lösungsmittel gelangen meist zusammen mit den vorgenannten Additiven und Co-Additiven, die sie zur besseren Handhabung lösen oder verdünnen sollen, in den Dieselkraftstoff.

**C) Kraftstoffe**

Das erfindungsgemäße Additiv eignet sich in hervorragender Weise als Kraftstoffzusatz und kann im Prinzip in jeglichen Kraftstoffen eingesetzt werden. Es bewirkt eine ganze Reihe von vorteilhaften Effekten beim Betrieb von Verbrennungsmotoren mit Kraftstoffen. Bevorzugt wird das erfindungsgemäße quaternisierte Additiv in Mitteldestillat-Kraftstoffen, insbesondere Dieselkraftstoffen, eingesetzt.

Gegenstand der vorliegenden Erfindung sind daher auch Kraftstoffe, insbesondere Mitteldestillat-Kraftstoffe, mit einem als Zusatzstoff zur Erzielung von vorteilhaften Effekten beim Betrieb von Verbrennungsmotoren, beispielsweise von Dieselmotoren, insbesondere von direkteinspritzenden Dieselmotoren, vor allem von Dieselmotoren mit Common-Rail-Einspritzsystemen, wirksamen Gehalt an dem erfindungsgemäßen quaternisierten Additiv. Dieser wirksame Gehalt (Dosierrate) liegt in der Regel bei 10 bis 5000 Gew.-ppm, vorzugsweise bei 20 bis 1500 Gew.-ppm, insbesondere bei 25 bis 1000 Gew.-ppm, vor allem bei 30 bis 750 Gew.-ppm, jeweils bezogen auf die Gesamtmenge an Kraftstoff.

Bei Mitteldestillat-Kraftstoffen wie Dieselkraftstoffen oder Heizölen handelt es sich vorzugsweise um Erdölraffinate, die üblicherweise einen Siedebereich von 100 bis 400°C haben. Dies sind meist Destillate mit einem 95%-Punkt bis zu 360°C oder auch darüber hinaus. Dies können aber auch so genannte "Ultra Low Sulfur Diesel" oder "City Diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.- %. Neben den durch Raffination erhältlichen mineralischen Mitteldestillat-Kraftstoffen bzw. Dieselkraftstoffen sind auch solche, die durch Kohlevergasung oder Gasverflüssigung ["gas to liquid" (GTL)-Kraftstoffe] oder durch Biomasse-Verflüssigung ["biomass to liquid" (BTL)-Kraftstoffe] erhältlich sind, geeignet. Geeignet sind auch Mischungen der vorstehend genannten Mitteldestillat-Kraftstoffe bzw. Dieselkraftstoffe mit regenerativen Kraftstoffen, wie Biodiesel oder Bioethanol.

Die Qualitäten der Heizöle und Dieselkraftstoffe sind beispielsweise in DIN 51603 und EN 590 näher festgelegt (vgl. auch Ullmann' s Encyclopedia of Industrial Chemistry, 5. Auflage, Band A12, S. 617 ff.).

Das erfindungsgemäße quaternisierte Additiv kann neben seiner Verwendung in den oben genannten Mitteldestillat-Kraftstoffen aus fossilem, pflanzlichem oder tierischem Ursprung, die im wesentlichen Kohlenwasserstoffmischungen darstellen, auch in Mischungen aus solchen Mitteldestillaten mit Biobrennstoffölen (Biodiesel) eingesetzt werden. Derartige Mischungen werden im Sinne der vorliegenden Erfindung auch von dem Begriff " Mitteldestillat-Kraftstoff" umfasst. Sie sind handelsüblich und enthalten meist die Biobrennstofföle in untergeordneten Mengen, typischerweise in Mengen von 1 bis 30 Gew.-% insbesondere von 3 bis 10 Gew.-%, bezogen auf die Gesamtmenge aus Mitteldestillat fossilen, pflanzlichem oder tierischen Ursprungs und Biobrennstofföl.

Biobrennstofföle basieren in der Regel auf Fettsäureestern, vorzugsweise im wesentlichen auf Alkylester von Fettsäuren, die sich von pflanzlichen und/oder tierischen Ölen und/oder Fetten ableiten. Unter Alkylestern werden üblicherweise Niedrigalkylester, insbesondere $C_1$- bis $C_4$-Alkylester, verstanden, die durch Umesterung der in pflanzlichen und/oder tierischen Ölen und/oder Fetten vorkommenden Glyceride, insbesondere Triglyceride, mittels Niedrigalkoholen, beispielsweise Ethanol oder vor allem Methanol (" FAME" ), erhältlich sind. Typische Niedrigalkylester auf Basis von pflanzlichen und/oder tierischen Ölen und/oder Fetten, die als Biobrennstofföl oder Komponenten hierfür Verwendung finden, sind beispielsweise Sonnenblumenmethylester, Palmölmethylester (" PME" ), Sojaölmethylester (" SME" ) und insbesondere Rapsölmethylester (" RME" ).

Besonders bevorzugt handelt es sich bei den Mitteldestillat-Kraftstoffen bzw. Dieselkraftstoffen um solche mit niedrigem Schwefelgehalt, das heißt mit einem Schwefelgehalt von weniger als 0,05 Gew.-%, vorzugsweise von weniger als 0,02 Gew.-%, insbesondere von weniger als 0,005 Gew.-% und speziell von weniger als 0,001 Gew.-% Schwefel.

Als Ottokraftstoffe kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

Das erfindungsgemäße quaternisierte Additiv eignet sich insbesondere als Kraftstoffzusatz in Kraftstoffzusammensetzungen, insbesondere in Dieselkraftstoffen, zur Überwindung der eingangs geschilderten Probleme bei direkteinspritzenden Dieselmotoren, vor allem bei solchen mit Common-Rail-Einspritzsystemen.
Die Erfindung wird nun anhand der folgenden Ausführungsbeispiele näher beschrieben. Insbesondere die im Folgenden genannten Testmethoden sind Teil der allgemeine Offenbarung der Anmeldung und nicht auf die konkreten Ausführungsbeispiele beschränkt.

**Experimenteller Teil:**

**Verwendete Reagenzien:**

N-Methyl-N,N-ditalgfettamin: Armeen® M2HT von Akzo Nobel, CAS 61788-63-4, Gesamtaminzahl 103-110 mg KOH/g.

Solvent Naphtha Heavy von Exxon Mobil, CAS 64742-94-5.
Dimethyloxalat von Aldrich, CAS 553-90-2
Laurinsäure von Aldrich, CAS 143-07-7
3,5,5-Trimethylhexansäure von BASF, CAS 3302-10-1
Methylsalicylat von Aldrich, CAS 119-36-8
2-Ethylhexanol von BASF, CAS 104-76-7
Essigsäure von Aldrich, CAS 64-19-7

**A. Allgemeine Testmethoden**

**Motorentest**

1. XUD9 Test - Bestimmung der Flow Restriction
Die Durchführung erfolgt nach den Standardbestimmungen gemäß CEC F-23-1-01.

2. DW10 Test - Bestimmung des Leistungsverlusts durch Injektorablagerungen im Common Rail Dieselmotor

**2.1. DW10- KC - Keep Clean Test**

Der Keep Clean Test lehnt sich an die CEC Test Prozedur F-098-08 Issue 5 an. Dabei kommen der gleiche Testaufbau und Motorentyp (PEUGEOT DW10 ) wie in der CEC Prozedur zum Einsatz.

**Änderung und Besonderheiten:**

Bei den Versuchen kamen gereinigte Injektoren zum Einsatz. Die Reinigungsdauer im Ultraschallbad in 60°C Wasser + 10% Superdecontamine (Intersciences, Brüssel) betrug 4h.

**Testlaufzeiten:**

Der Testzeitraum betrug 12h ohne Abstellphasen. Der in Figur 2 dargestellte einstündige Testzyklus aus der CEC F-098-08 wurde dabei 12-mal durchfahren.

**Leistungsbestimmung:**

Die Anfangsleistung P0,KC [kW] wird aus dem gemessenen Drehmoment bei 4000/min Volllast direkt nach Teststart und Warmlauf des Motors berechnet. Die Vorgehensweise ist in der Issue 5 der Testprozedur (CEC F-98-08) beschrieben. Dabei wird der gleiche Testaufbau und der Motorentyp PEUGEOT DW10 verwendet.
Die Endleistung (Pend,KC) wird im 12. Zyklus in Stufe 12, (siehe Tabelle, Figur 2) bestimmt. Auch hier ist der Betriebspunkt 4000/min Volllast. Pend,KC [kW] errechnet sich aus dem gemessenen Drehmoment.
Der Leistungsverlust im KC Test wird wie folgt berechnet:

$$\textbf{Powerloss ,KC [\%]} = \left(1 - \frac{Pend,KC}{P0,KC}\right) * 100$$

**2.2. DW10-Dirty Up - Clean Up-(DU-CU)**

Der DU-CU Test lehnt sich an die CEC Test Prozedur F-098-08 Issue 5 an. Die Vorgehensweise ist in der Issue 5 der Testprozedur (CEC F-98-08) beschrieben. Dabei wird der gleiche Testaufbau und der Motorentyp PEUGEOT DW10 verwendet.

Der DU - CU Test besteht aus zwei einzelnen Tests, die hintereinander gefahren werden. Der erste Test dient zur Ablagerungsbildung (DU), der zweite zum Entfernen der Ablagerungen (CU). Nach dem DU wird der Leistungsverlust (Powerloss) bestimmt. Nach Ende des DU Laufs wird der Motor für mindestens 8 Stunden nicht betrieben und auf Umgebungstemperatur abgekühlt. Danach wird mit dem CU Kraftstoff der CU gestartet, ohne die Injektoren auszubauen und zu reinigen. Die Ablagerungen und der powerloss gehen im Idealfall im CU-Testverlauf zurück.

**Änderung und Besonderheiten:**

Gereinigte Injektoren wurden vor jedem DU Test in den Motor eingebaut. Die Reinigungsdauer im Ultraschallbad bei 60°C Wasser + 10% Superdecontamine (Intersciences, Brüssel) betrug 4h.

**Testlaufzeiten:**

Der Testzeitraum betrug 12h für den DU und 12h für den CU. Der Motor wurde im DU und CU Test ohne Abstellphasen betrieben.

Der in Figur 2 dargestellte einstündige Testzyklus aus der CEC F-098-08 wurde dabei jeweils 12-mal durchfahren.

**Leistungsbestimmung:**

Die Anfangsleistung P0,du [kW] wird aus dem gemessenen Drehmoment bei 4000/min Volllast direkt nach Teststart und Warmlauf des Motors berechnet. Die Vorgehensweise ist ebenfalls in der Issue 5 der Testprozedur beschrieben.

Die Endleistung (Pend,du) wird im 12. Zyklus in Stufe 12, (siehe Tabelle oben) bestimmt. Auch hier ist der Betriebspunkt 4000/min Volllast. Pend,du [kW] errechnet sich aus dem gemessenen Drehmoment.

Der Leistungsverlust im DU wird wie folgt berechnet

$$\textbf{Powerloss,du [\%]} = \left(1 - \frac{Pend,du}{P0,du}\right) * 100$$

Clean up

Die Anfangsleistung **P0,cu** [kW] wird aus dem gemessenen Drehmoment bei 4000/min Volllast direkt nach Teststart und Warmlauf des Motors im CU berechnet. Die Vorgehensweise ist ebenfalls in der Issue 5 der Testprozedur beschrieben.

Die Endleistung (Pend,cu) wird im 12. Zyklus in Stufe 12, (siehe Tabelle Figur 2) bestimmt. Auch hier ist der Betriebspunkt 4000/min Volllast. Pend,cu [kW] errechnet sich aus dem gemessenen Drehmoment.

Der Leistungsverlust im CU-Test wird wie folgt berechnet (negative Zahl beim powerloss im cu-Test bedeutet Leistungszuwachs)

$$\textbf{Powerloss (DU,CU)[\%]} = \left(\frac{Pend,du - pend,cu}{P0,du}\right) * 100$$

Als Kraftstoff wurde ein handelsüblicher Dieselkraftstoff der Fa. Haltermann (RF-06-03) eingesetzt. Diesem wurden zur künstlichen Anregung der Bildung von Ablagerungen an den Injektoren 1 Gew.-ppm Zink in Form einer Zink-Didodecanoat-Lösung zugesetzt.

### 3. IDID Test - Bestimmung der Additivwirkung gegen interne Injektorablagerungen

Die Bildung von Ablagerungen im Inneren des Injektors wurde anhand der Abweichungen der Abgastemperaturen der Zylinder am Zylinderausgang beim Kaltstart des DW10-Motors charakterisiert.

Zur Förderung der Bildung von Ablagerungen wurden dem Kraftstoff 1 mg/l Na Salz einer organischen Säure, 20 mg/l Dodecenylbernsteinsäure und 10 mg/l Wasser zugegeben.

Der Test wird als dirty-up-clean-up Test (DU-CU) durchgeführt.

DU-CU lehnt sich an die an die CEC Test Prozedur F-098-08 Issue 5 an.

Der DU - CU Test besteht aus zwei einzelnen Tests, die hintereinander gefahren werden. Der erste Test dient zur Ablagerungsbildung (DU), der zweite zum Entfernen der Ablagerungen (CU).

Nach dem DU Lauf wird nach einer mindestens achtstündigen Stillstands-Phase ein Kaltstart des Motors mit anschließendem 10-minütigen Leerlauf durchgeführt.

Danach wird mit dem CU Kraftstoff der CU gestartet, ohne die Injektoren auszubauen und zu reinigen. Nach dem CU Lauf über 8h wird nach einer mindestens achtstündigen Stillstands-Phase ein Kaltstart des Motors mit anschließendem 10-minütigen Leerlauf durchgeführt. Die Auswertung erfolgt durch den Vergleich der Temperaturverläufe für die einzelnen Zylinder nach Kaltstart des du und des CU-Laufs.

Der IDID-Test zeigt die interne Ablagerungsbildung im Injektor an. Als Kenngröße dient bei diesem Test die Abgastemperatur der einzelnen Zylinder. Bei einem Injektorsystem ohne IDID erhöhen sich die Abgastemperaturen der Zylinder gleichmäßig. Bei vorhandenem IDID erhöhen sich die Abgastemperaturen der einzelnen Zylinder nicht gleichmäßig und weichen voneinander ab.

Die Temperatursensoren befinden sich hinter dem Zylinderkopfaustritt im Abgaskrümmer. Signifikante Abweichung der einzelnen Zylindertemperaturen (z.B. > 20°C) zeigen das Vorliegen von internen Injektorablagerungen (IDID) an.

Die Tests (DU und CU) werden mit jeweils 8h Laufzeit durchgeführt. Der einstündige Testzyklus aus der CEC F-098-08 (siehe Figur 3) wird dabei jeweils 8-mal durchfahren. Bei Abweichungen der einzelnen Zylindertemperaturen von größer 45°C zum Mittelwert aller 4 Zylinder wird der Test vorzeitig abgebrochen.

Änderung und Besonderheiten: Gereinigte Injektoren wurden vor jedem DU-Testbeginn eingebaut. Die Reinigungsdauer im Ultraschallbad bei 60°C Wasser + 10% Superdecontamine betrug 4h.

## B. Herstellungsbeispiele:

### Herstellungsbeispiel 1: N,N-Dimethyl-N,N-ditalgfettammoniummethyloxalat wurde in Anlehnung an EP 2 033 945 synthetisiert

N-Methyl-N,N-ditalgfettamin (90 g) wird mit Dimethyloxalat (90 g) und Laurinsäure (1,8 g) versetzt. Das Reaktionsgemisch wird auf 120°C erhitzt und 4 h bei dieser Temperatur gerührt. Anschließend wird überschüssiges Dimethyloxalat mit Hilfe eines Rotationsverdampfers bei 130°C im Vakuum entfernt. Man erhält 110,8 g des Produktes als weißes Wachs. $^1$H-NMR ($CDCl_3$) bestätigt die Quaternierung.

### Herstellungsbeispiel 2: N,N-Dimethyl-N,N-ditalgfettammoniumsalicylat

N-Methyl-N,N-ditalgfettamin (80 g) wird mit Methylsalicylat (45,4 g) und 3,5,5-Trimethylhexansäure (0,8 g) versetzt. Das Reaktionsgemisch wird auf 160°C erhitzt und 4 h bei dieser Temperatur gerührt. Nach Abkühlen auf Raumtemperatur erhält man 124 g des Produktes als weißes Wachs. $^1$H-NMR ($CDCl_3$) bestätigt die Quaternierung.

### Herstellungsbeispiel 3: N-Methyl-N-(2-hydroxypropyl)-N,N-ditalgfettammoniumacetat

In einem 2 l Autoklaven wird eine Lösung von N-Methyl-N,N-ditalgfettamin (250 g) in 2-Ethylhexanol (250 g) mit Essigsäure (100%, 33,5 g) versetzt. Anschließend wird dreimal mit $N_2$ gespült, ein Vordruck von ca. 1,3 bar $N_2$ eingestellt und die Temperatur auf 50°C erhöht. Propylenoxid (54 g) wird so zudosiert, dass die Temperatur zwischen 45-55°C bleibt. Anschließend wird 10 h bei 50°C nachgerührt, auf 25°C abgekühlt, mit $N_2$ gespült und der Reaktor entleert. Das Produkt wird für 3 h bei 80°C und 20 mbar am Rotationsverdampfer entgast. Man erhält 549,4 g des Produktes in 2-Ethylhexanol. $^1$H-NMR ($CDCl_3$) bestätigt die Quaternierung. Die Probe wird durch Zugabe von Solvent Naphtha Heavy auf einen Wirkstoffgehalt von 38% eingestellt.

## C. Anwendungsbeispiele:

In den folgenden Anwendungsbeispielen werden die Additive entweder als Reinsubstanz (so wie in obigen

Herstellungsbeispielen synthetisiert) oder in Form eines Additiv-Paketes eingesetzt.

**Anwendungsbeispiel 1: Bestimmung der Additivwirkung auf die Bildung von Ablagerungen in Dieselmotor-Einspritzdüsen**

a) XUD9 Tests

Verwendeter Kraftstoff: RF-06-03 (Referenzdiesel, Haltermann Products, Hamburg)

Die Ergebnisse sind in Tabelle 1 zusammengefasst.

Tabelle 1: Ergebnisse der XUD9 Tests

| Bsp. | Bezeichnung | Dosierung ppm active | Flow restriction 0,1 mm Nadelhub [%] |
|---|---|---|---|
| #1 | gemäß Herstellungsbeispiel 1 | 30 | 8.4 |
| #2 | gemäß Herstellungsbeispiel 1 | 15 | 22.4 |

b) DW10 Test

Die nachfolgende Tabelle zeigt die Ergebnisse der Bestimmungen des relativen Leistungsverlustes (Power loss) bei 4000 rpm nach 12 Stunden Dauerbetrieb ohne Unterbrechung. Der Wert P0 gibt dabei die Leistung nach 10 Minuten und der Wert Pend die Leistung am Ende der Messung an:
Die Versuchsergebnisse sind in Tabelle 2 dargestellt.

Tabelle 2: Ergebnisse des DW10 Tests

| Additiv | Dosis [mg/kg] | Powerloss KC | Powerloss DU | Powerloss DU-CU |
|---|---|---|---|---|
| Grundwert | 0 | | 4,1% | |
| gemäß Herstellungsbeispiel 1, Keep clean | 100 | 0.4 % | | |
| gemäß Herstellungsbeispiel 1, Clean-up | 100 | | | -4.9% |
| Grundwert | 0 | | 3.8% | |
| gemäß Herstellungsbeispiel 2 Keep Clean | 100 | -0.4 % | | |

Dabei zeigt sich, dass die erfindungsgemäßen Additive gemäß Herstellungsbeispiel 1 und 2 eine gegenüber dem Grundwert verbesserte Wirkung aufweisen

c) Wirkung gegen interne Injektorablagerungen (IDID)

Verwendeter Kraftstoff: RF-06-03 (Referenzdiesel, Haltermann Products, Hamburg)

Die Versuchsergebnisse sind in beiliegenden Figuren 1 und 2 dargestellt.

Figur 1 zeigt eine Messung der Abgastemperaturen der Zylinder bei Verwendung eines Kraftstoffs ohne Additiv; hohe Abweichungen der Temperatur werden durch interne Injektorablagerungen verursacht

Figur 2 zeigt die gemessenen Abgastemperaturen derselben Zylinder nach Behandlung mit dem erfindungsgemäßen Additiv aus Herstellbeispiel 3, Dosierung 394 mg/kg.

Die Messungen veranschaulichen die Wirkung des erfindungsgemäßen Additivs zur Auflösung von internen Injektorablagerungen. Die durch die internen Injektorablagerungen verursachten Senkungen der Abgastemperatur (Fig. 1, Zylinder 1 und 4) können durch das erfindungsgemäße Additiv wieder aufgehoben werden.

Auf die Offenbarung der hierin zitierten Druckschriften wird ausdrücklich Bezug genommen.

## Patentansprüche

**1.** Verwendung eines eine quaternisierte Stickstoffverbindung umfassenden Reaktionsprodukts oder einer aus dem Reaktionsprodukt durch Aufreinigung erhaltene, eine quaternisierte Stickstoffverbindung enthaltende Teilfraktion davon, wobei das Reaktionsprodukt erhältlich ist durch

Umsetzung eines quaternisierbaren Alkylamins, enthaltend wenigstens eine quaternisierbare, tertiäre Aminogruppe mit einem Quaternierungsmittel, das die wenigstens eine tertiäre Aminogruppe in eine quaternäre Ammoniumgruppe überführt,
wobei das Quaternierungsmittel eine Verbindung der allgemeinen Formel 1 ist,

$R_1OC(O)R_2$ (1)

worin
$R_1$ für einen gesättigten, geradkettigen oder verzweigten Kohlenwasserstoffrest mit 1 bis 4 Kohlenstoffatomen steht und
$R_2$ für einen gegebenenfalls substituierten einkernigen Aryl- oder Cycloalkylrest steht, wobei der Substituent ausgewählt ist unter OH, $NH_2$, $NO_2$, C(O)ORs und $R_1OC(O)$-; worin $R_1$ die oben angegebenen Bedeutungen besitzt und $R_3$ für H oder $R_1$ steht;
oder $R_2$ für $R_{1a}OC(O)$- steht, worin $R_{1a}$ die oben für $R_1$ angegebenen Bedeutungen besitzt; oder
wobei das Quaternierungsmittel eine Verbindung der allgemeinen Formel 2 ist,

$R_1OC(O)\text{-}A\text{-}C(O)OR_{1a}$ (2)

worin
$R_1$ und $R_{1a}$ unabhängig voneinander für einen gesättigten, geradkettigen oder verzweigten Kohlenwasserstoffrest mit 1 bis 4 Kohlenstoffatomen steht und
A für eine gegebenenfalls ein- oder mehrfach substituierte geradkettige oder ein- oder mehrfach verzweigte Kohlenwasserstoffbrückengruppe mit 1-10 Kohlenstoffatomen steht, welche gegebenenfalls durch ein oder mehrere Heteroatomgruppen unterbrochen ist;
oder
wobei das Quaternisierungsmittel ein Epoxid der allgemeinen Formel 4 ist,

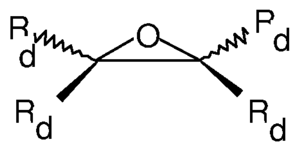

(4)

wobei
die darin enthaltenen Reste $R_d$ gleich oder verschieden sind und für H oder für einen aliphatischen oder aromatischen Rest mit wenigstens 1 bis 10 Kohlenstoffatomen stehen, wobei die aliphatischen Reste $R_d$ zusätzlich Heteroatome, wie O, N, NH, S, in ihrer Kette enthalten können; alleine oder in Kombination mit einer freien Säure umfasst; oder
wobei das Quaternisierungsmittel ein Dialkylcarbonat ist;
und wobei
das Alkylamin wenigstens eine Verbindung der folgenden allgemeinen Formel 3 umfasst,

$R_aR_bR_cN$ (3)

worin
wenigstens einer der Reste $R_a$, $R_b$ und $R_c$ für einen geradkettigen oder verzweigte, gesättigten oder ungesättigten $C_8$-$C_{40}$-Kohlenwasserstoffrest, welcher keine Heteroatome aufweist, steht und die übrigen Reste für gleiche oder verschiedene, geradkettige oder verzweigte, gesättigte oder ungesättigte $C_1$-$C_6$-Kohlenwasserstoffreste stehen; oder
worin alle Reste $R_a$, $R_b$ und $R_c$ für gleiche oder verschiedene geradkettige oder verzweigte, gesättigte oder ungesättigte $C_8$-$C_{40}$-Kohlenwasserstoffreste, welche keine Heteroatome aufweisen, stehen,
als Dieselkraftstoffadditiv zur Verringerung und/oder Vermeidung von Internal Diesel Injector Deposits (IDID)in

direkteinspritzenden Dieselmotoren.

2. Verwendung nach Anspruch 1, wobei A für ein gegebenenfalls ein- oder mehrfach substituiertes Alkylen oder Alkenylen steht.

3. Verwendung nach einem der Ansprüche 1 und 2, wobei $R_1$ in Verbindungen der Formel 1 für Methyl oder Ethyl steht; und
$R_1$ und $R_{1a}$ in Verbindungen der Formel 2 unabhängig voneinander für Methyl oder Ethyl stehen.

4. Verwendung nach Anspruch einem der vorhergehenden Ansprüche als Dieselkraftstoffadditiv in Dieselmotoren mit Common-Rail-Einspritzsystemen.

5. Verwendung nach einem der vorhergehenden Ansprüche, wobei das Quaternierungsmittel ein Epoxid der allgemeinen Formel 4 umfasst

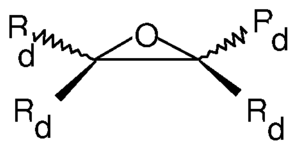

(4)

wobei
die darin enthaltenen Reste $R_d$ gleich oder verschieden sind und für H oder für einen aliphatischen oder aromatischen Rest mit wenigstens 1 bis 10 Kohlenstoffatomen stehen, wobei die aliphatischen Reste $R_d$ zusätzlich Heteroatome, wie O, N, NH, S, in ihrer Kette enthalten können; und die freie Säure des Quaternierungsmittels eine freie Protonensäure ist.

6. Verwendung nach Anspruch 5, wobei die freie Protonensäure eine $C_{1-12}$-Monocarbonsäure oder -Dicarbonsäure ist.

7. Verwendung nach einem der vorhergehenden Ansprüche, wobei das quaternisierbare tertiäre Amin eine Verbindung der Formel 3 ist, worin wenigstens zwei der Reste $R_a$, $R_b$ und $R_c$ gleich oder verschieden sind und für einen geradkettigen oder verzweigte, $C_{10}$-$C_{20}$-Alkylrest steht und der übrigen Rest für $C_1$-$C_4$-Alkyl steht.

8. Verwendung nach einem der vorhergehenden Ansprüche, wobei das Quaternierungsmittel ausgewählt ist unter $C_2$-$C_{12}$-Alkylenoxiden in Kombination mit einer Monocarbonsäure, Alkylsalicylaten, Dialkylphthalaten und Dialkyloxalaten.

9. Verwendung nach einem der vorhergehenden Ansprüche, wobei der Kraftstoff ausgewählt ist unter Dieselkraftstoffen und Biodieselkraftstoffen.

10. Verwendung nach einem der vorhergehenden Ansprüche, wobei das Quaternisierungsmittel ein Dialkylcarboxylat ist.

11. Verwendung nach Anspruch 10, wobei das Dialkylcarboxylat ein Dialkylphtalat oder ein Dialkyloxalat ist.

**Claims**

1. The use of a reaction product comprising a quaternized nitrogen compound, or of a fraction thereof which comprises a quaternized nitrogen compound and is obtained from the reaction product by purification, wherein the reaction product is obtainable by

reacting a quaternizable alkylamine comprising at least one quaternizable tertiary amino group with a quaternizing agent which converts the at least one tertiary amino group to a quaternary ammonium group, wherein the quaternizing agent is a compound of the general formula 1

$$R_1OC(O)R_2 \quad (1)$$

in which
$R_1$ is a saturated, straight-chain or branched hydrocarbyl radical having 1 to 4 carbon atoms and $R_2$ is an optionally substituted monocyclic aryl or cycloalkyl radical, where the substituent is selected from OH, $NH_2$, $NO_2$, $C(O)OR_3$, and $R_1OC(O)$-; in which $R_2$ is as defined above and $R_3$ is H or $R_1$;
or $R_2$ is $R_{1a}OC(O)$- in which $R_{1a}$ has the definitions given above for $R_1$; or
wherein the quaternizing agent is a compound of the general formula 2

$$R_1OC(O)\text{-}A\text{-}C(O)OR_{1a} \qquad (2)$$

in which
$R_1$ and $R_{1a}$ are each independently a saturated, straight-chain or branched hydrocarbyl radical having 1 to 4 carbon atoms and
A is an optionally mono- or polysubstituted, straight-chain or singly or multiply branched hydrocarbylene bridging group which has 1-10 carbon atoms and may optionally be interrupted by one or more heteroatom groups;
or
wherein the quaternizing agent is an epoxide of the general formula 4

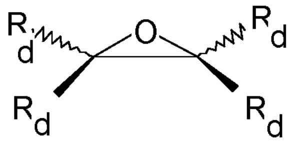

(4)

where
the $R_d$ radicals present therein are the same or different and are each H or an aliphatic or aromatic radical having at least 1 to 10 carbon atoms, where the aliphatic radicals $R_d$ may additionally contain heteroatoms, such as O, N, NH, S, in their chain; alone or in combination with a free acid; or wherein the quaternizing agent is a dialkyl carbonate;
and wherein
the alkylamine comprises at least one compound of the following general formula 3

$$R_aR_bR_cN \qquad (3)$$

in which
at least one of the $R_a$, $R_b$ and $R_c$ radicals is a straight-chain or branched, saturated or unsaturated $C_8$-$C_{40}$-hydrocarbyl radical which does not have any heteroatoms and the remaining radicals are identical or different, straight-chain or branched, saturated or unsaturated $C_1$-$C_6$-hydrocarbyl radicals; or
in which all $R_a$, $R_b$ and $R_c$ radicals are identical or different, straight-chain or branched, saturated or unsaturated $C_8$-$C_{40}$-hydrocarbyl radicals which do not have any heteroatoms;
as a diesel fuel additive for reduction and/or prevention of internal diesel injector deposits (IDIDs) in direct injection diesel engines.

2. The use according to claim 1, wherein A is an optionally mono- or polysubstituted alkylene or alkenylene.

3. The use according to either of claims 1 and 2, wherein $R_1$ in compounds of the formula 1 is methyl or ethyl; and $R_1$ and $R_{1a}$ in compounds of the formula 2 are independently methyl or ethyl.

4. The use according to claim any of the preceding claims as diesel fuel additive in diesel engines with common rail injection systems.

5. The use according to any of the preceding claims, wherein the quaternizing agent comprises an epoxide of the general formula 4

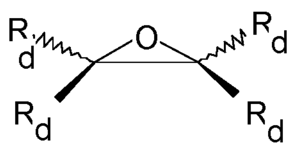

(4)

where the $R_d$ radicals present therein are the same or different and are each H or an aliphatic or aromatic radical having at least 1 to 10 carbon atoms, where the aliphatic radicals $R_d$ may additionally contain heteroatoms, such as O, N, NH, S, in their chain; and the free acid of the quaternizing agent is a free protic acid.

**6.** The use according to claim 5, wherein the free protic acid is a $C_{1-}C_{12}$ monocarboxylic acid or dicarboxylic acid.

**7.** The use according to any of the preceding claims, wherein the quaternizable tertiary amine is a compound of the formula 3 in which at least two of the $R_a$, $R_b$ and $R_c$ radicals are the same or different and are each a straight-chain or branched $C_{10}$-$C_{20}$-alkyl radical and the other radical is $C_1$-$C_4$-alkyl.

**8.** The use according to any of the preceding claims, wherein the quaternizing agent is selected from $C_2$-$C_{12}$-alkylene oxides in combination with a monocarboxylic acid, alkyl salicylates, dialkyl phthalates and dialkyl oxalates.

**9.** The use according to any of the preceding claims, wherein the fuel is selected from diesel fuels and biodiesel fuels.

**10.** The use according to any of the preceding claims, wherein the quaternizing agent is a dialkyl carboxylate.

**11.** The use according to claim 10, wherein the dialkyl carboxylate is a dialkyl phthalate or a dialkyl oxalate.

**Revendications**

**1.** Utilisation d'un produit de réaction comprenant un composé azoté quaternisé ou d'une fraction partielle de celui-ci, obtenue à partir du produit de réaction par purification, contenant un composé azoté quaternisé, le produit de réaction pouvant être obtenu par transformation d'une alkylamine quaternisable, contenant au moins un groupe amino tertiaire quaternisable, par un agent de quaternisation, qui transforme ledit au moins un groupe amino tertiaire en un groupe d'ammonium quaternaire,

l'agent de quaternisation étant un composé de formule générale 1,

$$R_1OC(O)R_2 \qquad (1)$$

dans laquelle

$R_1$ représente un radical hydrocarboné saturé, linéaire ou ramifié comprenant 1 à 4 atomes de carbone et $R_2$ représente un radical aryle ou cycloalkyle à un noyau, le cas échéant substitué, le substituant étant choisi parmi OH, $NH_2$, $NO_2$, $C(O)OR_3$, et $R_1OC(O)$- ; $R_1$ présentant les significations indiquées ci-dessus et $R_3$ représentant H ou $R_1$ ; ou
$R_2$ représente $R_{1a}OC(O)$ -, où $R_{1a}$ présente les significations indiquées ci-dessus pour $R_1$ ; ou

l'agent de quaternisation étant un composé de formule générale 2,

$$R_1OC(O)\text{-}A\text{-}C(O)OR_{1a} \qquad (2)$$

dans laquelle

$R_1$ et $R_{1a}$ représentent, indépendamment l'un de l'autre, un radical hydrocarboné saturé, linéaire ou ramifié, comprenant 1 à 4 atomes de carbone et
A représente un groupe formant un pont hydrocarboné, le cas échéant monosubstitué ou polysubstitué, linéaire ou monoramifié ou polyramifié, monoinsaturé ou polyinsaturé, comprenant 1-10 atomes de carbone, qui est le cas échéant interrompu par un ou plusieurs groupes hétéroatomiques ; ou

l'agent de quaternisation étant un époxyde de formule générale 4,

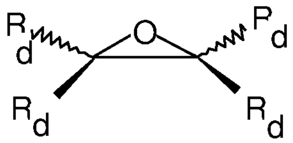

(4)

dans laquelle les radicaux $R_d$ qui y sont contenus sont identiques ou différents et représentent H ou un radical aliphatique ou aromatique comprenant au moins 1 à 10 atomes de carbone, les radicaux $R_d$ aliphatiques pouvant en outre contenir des hétéroatomes, tels que O, N, NH, S, dans leur chaîne ; seul ou en combinaison avec un acide libre ; ou
l'agent de quaternisation étant un carbonate de dialkyle ; et
l'alkylamine comprenant au moins un composé de la formule générale 3 suivante,

$R_aR_bR_cN$ (3)

dans laquelle
au moins l'un des radicaux $R_a$, $R_b$ et $R_c$ représente un radical hydrocarboné en $C_8$-$C_{40}$, linéaire ou ramifié, saturé ou insaturé, qui ne présente pas d'hétéroatomes et les autres radicaux représentent des radicaux hydrocarbonés en $C_1$-$C_6$ identiques ou différents, linéaires ou ramifiés, saturés ou insaturés ; ou
tous les radicaux $R_a$, $R_b$ et $R_c$ représentent des radicaux hydrocarbonés en $C_8$-$C_{40}$ identiques ou différents, linéaires ou ramifiés, saturés ou insaturés, qui ne présentent pas d'hétéroatomes,
comme additif à un carburant de type diesel pour diminuer et/ou éviter des dépôts internes des injecteurs diesel (Internal Diesel Injector Deposits - IDID) dans des moteurs diesel à injection directe.

**2.** Utilisation selon la revendication 1, A représentant un alkylène ou un alcénylène le cas échéant monosubstitué ou polysubstitué.

**3.** Utilisation selon l'une quelconque des revendications 1 et 2, où, dans les composés de formule 1, $R_1$ représente méthyle ou éthyle ; et
dans les composés de formule 2, $R_1$ et $R_{1a}$ représentent, indépendamment l'un de l'autre, méthyle ou éthyle.

**4.** Utilisation selon la revendication l'une quelconque des revendications précédentes comme additif pour carburant de type diesel dans des moteurs diesel présentant des systèmes d'injection à rampe commune.

**5.** Utilisation selon l'une quelconque des revendications précédentes, l'agent de quaternisation comprenant un époxyde de formule générale 4

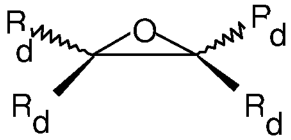

(4)

dans laquelle
les radicaux $R_d$ qui y sont contenus sont identiques ou différents et représentent H ou un radical aliphatique ou aromatique comprenant au moins 1 à 10 atomes de carbone, les radicaux $R_d$ aliphatiques pouvant en outre contenir des hétéroatomes, tels que O, N, NH, S, dans leur chaîne ; et l'acide libre de l'agent de quaternisation étant un acide protonique libre

**6.** Utilisation selon la revendication 5, l'acide protonique libre étant un acide monocarboxylique ou dicarboxylique en $C_{1-12}$.

**7.** Utilisation selon l'une quelconque des revendications précédentes, l'amine tertiaire quaternisable étant un composé de formule 3, au moins deux des radicaux $R_a$, $R_b$ et $R_c$ étant identiques ou différents et représentant un radical $C_{10}$-$C_{20}$-alkyle linéaire ou ramifié et l'autre radical représentant $C_1$-$C_4$-alkyle.

**8.** Utilisation selon l'une quelconque des revendications précédentes, l'agent de quaternisation étant choisi parmi les oxydes de $C_2$-$C_{12}$-alkylène en combinaison avec un acide monocarboxylique, des salicylates d'alkyle, des phtalates

de dialkyle et des oxalates de dialkyle.

**9.** Utilisation selon l'une quelconque des revendications précédentes, le carburant étant choisi parmi les carburants de type diesel et les carburants de type biodiesel.

**10.** Utilisation selon l'une quelconque des revendications précédentes, l'agent de quaternisation étant un carbonate de dialkyle.

**11.** Utilisation selon la revendication 10, le carboxylate de dialkyle étant un phtalate de dialkyle ou un oxalate de dialkyle.

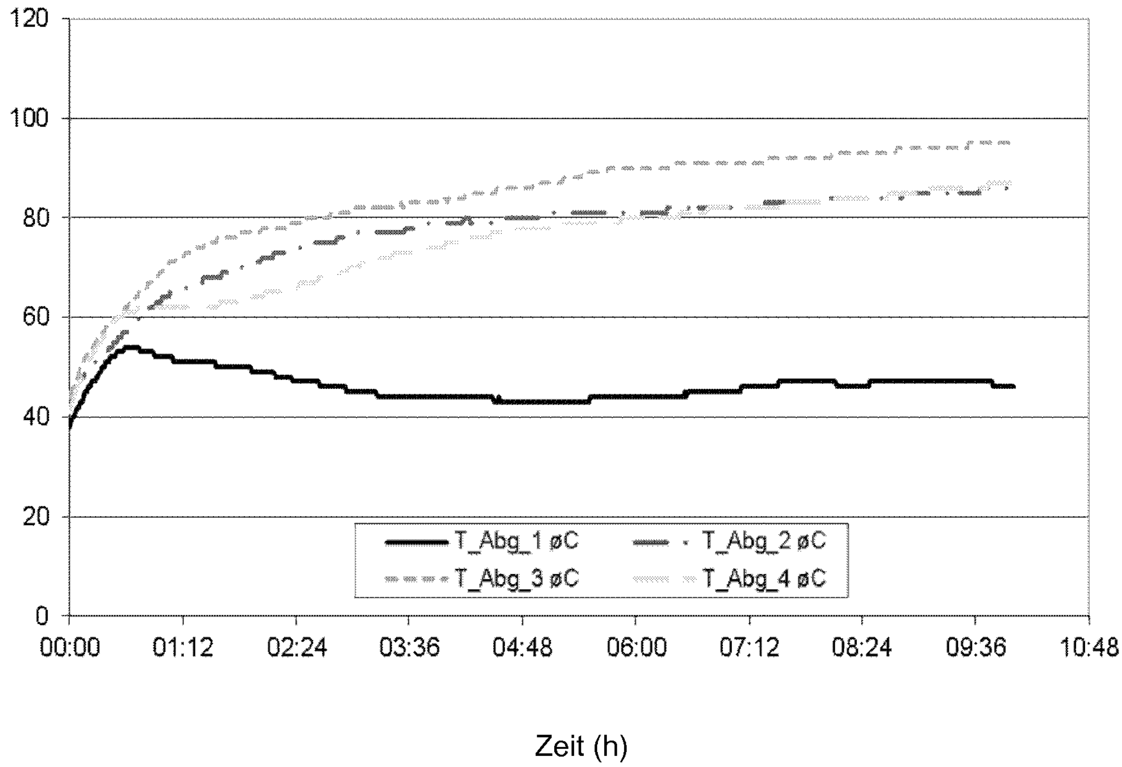
120
100
80
60
40
20
0
00:00
01:12
02:24
03:36
04:48
06:00
07:12
08:24
09:36
10:48
T_Abg_1 øC
T_Abg_2 øC
T_Abg_3 øC
T_Abg_4 øC
Zeit (h)

**Fig.1**

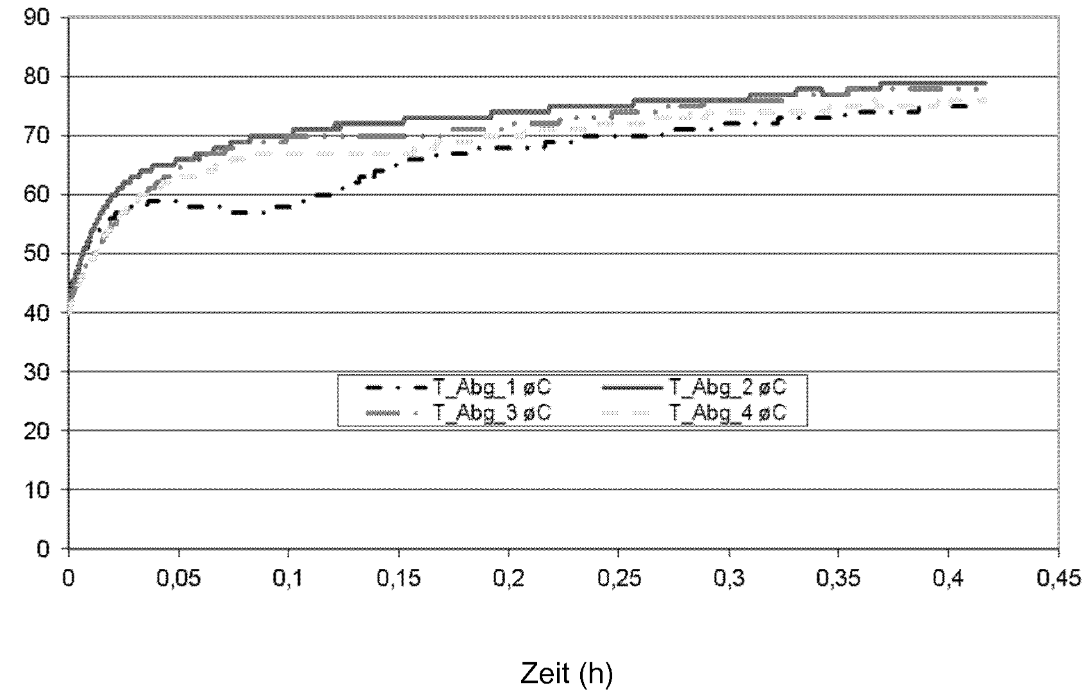
90
80
70
60
50
40
30
20
10
0
0
0,05
0,1
0,15
0,2
0,25
0,3
0,35
0,4
0,45
T_Abg_1 øC
T_Abg_2 øC
T_Abg_3 øC
T_Abg_4 øC
Zeit (h)

**Fig.2**

| step | duration (minutes) | engine speed (rpm) +/- 20 | load (%) | torque (Nm) +/-5 | boost air after IC (°C) +/-3 |
|---|---|---|---|---|---|
| 1 | 2' | 1750 | (20) | 62 | 45 |
| 2 | 7' | 3000 | (60) | 173 | 50 |
| 3 | 2' | 1750 | (20) | 62 | 45 |
| 4 | 7' | 3500 | (80) | 212 | 50 |
| 5 | 2' | 1750 | (20) | 62 | 45 |
| 6 | 10' | 4000 | 100 | * | 50 |
| 7 | 2' | 1250 | (10) | 25 | 43** |
| 8 | 7' | 3000 | 100 | * | 50 |
| 9 | 2' | 1250 | (10) | 25 | 43** |
| 10 | 10' | 2000 | 100 | * | 50 |
| 11 | 2' | 1250 | (10) | 25 | 43** |
| 12 | 7' | 4000 | 100 | * | 50 |
| | Σ= 1 hour | | | | |

* for expected range see appendix 06.5

** target only

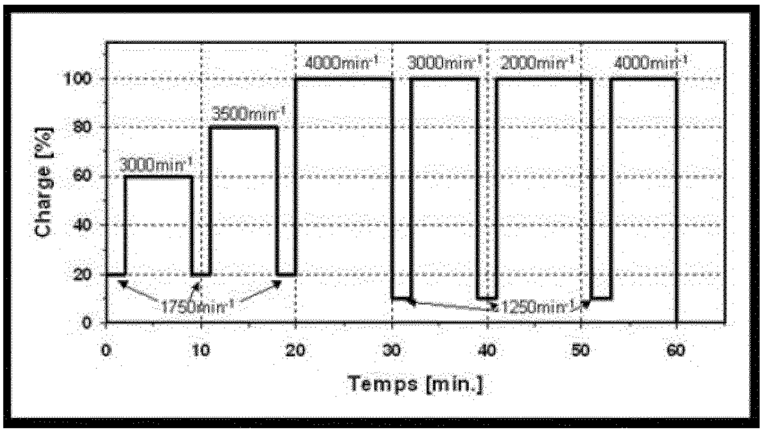


Fig.3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4248719 A
- US 4171959 A
- EP 2033945 A
- WO 2006135881 A
- EP 1254889 A
- WO 2010132259 A
- EP 244616 A
- WO 9424231 A
- WO 9703946 A
- DE 19620262 A
- WO 9603367 A
- WO 9603479 A
- EP 476485 A
- EP 307815 A
- WO 8701126 A
- EP 639632 A
- EP 310875 A
- EP 356725 A
- EP 700985 A
- US 4877416 A
- DE 3838918 A
- EP 831141 A
- DE 3826608 A
- DE 4142241 A
- DE 4309074 A
- EP 452328 A
- EP 548617 A
- DE 10102913 A
- WO 9929748 A
- WO 2005054314 A
- WO 2004035715 A
- EP 061895 A
- US 4491455 A
- WO 9318115 A
- EP 261957 A
- WO 0044857 A
- WO 98004656 A
- US 6743266 B2
- WO 0047698 A


### In der Beschreibung aufgeführte Nicht-Patentliteratur


- **N. A. PLATÉ ; V. P. SHIBAEV.** Comb-Like Polymers. Structure and Properties. *J. Poly. Sci. Macromolecular Revs.,* 1974, vol. 8, 117-253
- Ullmann' s Encyclopedia of Industrial Chemistry. vol. A12, 617
- *CHEMICAL ABSTRACTS,* 61788-63-4
- *CHEMICAL ABSTRACTS,* 64742-94-5
- *CHEMICAL ABSTRACTS,* 553-90-2
- *CHEMICAL ABSTRACTS,* 143-07-7
- *CHEMICAL ABSTRACTS,* 3302-10-1
- *CHEMICAL ABSTRACTS,* 119-36-8
- *CHEMICAL ABSTRACTS,* 104-76-7
- *CHEMICAL ABSTRACTS,* 64-19-7